Europäisches Patentamt
European Patent Office
Office européen des brevets

(11) Publication number: **0 002 881 A1**

# (12) EUROPEAN PATENT APPLICATION

(21) Application number: **78300497.1**

(22) Date of filing: **12.10.78**

(51) Int. Cl.[2]: **C 07 D 261/14**, A 01 N 5/00, C 07 D 261/18, A 01 N 9/22 // C07D413/04, C07D413/06, C07D417/04

(30) Priority: 02.11.77 GB 4555577

(43) Date of publication of application: **11.07.79 Bulletin 79/14**

(84) Designated Contracting States: **BE CH DE FR GB LU NL SE**

(71) Applicant: **FISONS LIMITED, Fison House 9 Grosvenor Street, London (GB)**

(72) Inventor: **Percival, Albert, Northumbria The Lane, Hauxton Cambridgeshire (GB)**
Inventor: **Rowson, Graham Pont, 8 Pilgrims Close Great Chesterford, Saffron Walden Essex (GB)**

(74) Representative: **Murphy, Finnbarr et al, Fisons Limited Fison House Princes Street, Ipswich 1P1 1QH (GB)**

## (54) 5-Isoxazolylurea derivatives, their preparation, their use and compositions containing them.

(57) 5-Isoxazalyl ureas of formula

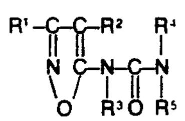

I

and salts thereof, wherein

$R^1$ represents a hydrogen or halogen atom or a nitro, amino, substituted amino, alkyl, substituted alkyl, cycloalkyl, aryl, substituted aryl, aralkyl, or substituted aralkyl, group, or a group of formula $-XR^6$ or $-COOR^{15}$ in which X represents oxygen, sulphur, sulphinyl or sulphonyl, $R^6$ represents alkyl or aryl, and $R^{15}$ represents a hydrogen atom or an alkyl, substituted alkyl, aryl, substituted aryl, or cycloalkyl, group;

$R^2$ represents cyano, nitro, carboxy, carboxy ester, carboxyamide, carboxyamide whose amide group is substituted, or a group of formula 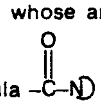 in which N) represents a 5–7 ring membered heterocyclic radical which optionally may be substituted;

$R^3$ represents hydrogen, alkyl or a group of formula 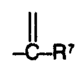 in which $R^7$ represents alkyl, aryl, alkoxy or alkylthio;

$R^4$ represents hydrogen, alkyl or cycloalkyl;

$R^5$ represents hydrogen, alkyl, cycloalkyl, alkoxy, aryl or substituted aryl; or

$R^3$ and $R^5$ together represent alkylene, optionally unsaturated, optionally interrupted by one or more atoms selected from nitrogen, oxygen, and sulphur atoms, and optionally substituted, are new compounds, which are pesticides or plant growth regulants, particularly selective herbicides.

EP 0 002 881 A1

HETEROCYCLIC COMPOUNDS, THEIR PRODUCTION, THEIR USE AND COMPOSITIONS CONTAINING THEM.

This invention relates to new compounds, their production, their pesticidal or plant growth regulant use and pesticidal or plant growth regulant compositions containing them.

The invention provides a compound which is a 5-isoxazolyl urea of formula

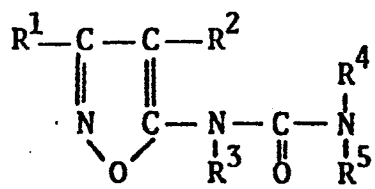

I

or a salt thereof,

wherein $R^1$ represents a hydrogen or halogen atom or a nitro, amino, substituted amino, alkyl, substituted alkyl, cycloalkyl, aryl, substituted aryl, aralkyl, or substituted aralkyl, group, or a group of formula $-XR^6$ or $-COOR^{15}$ in which X represents oxygen, sulphur, sulphinyl or sulphonyl, $R^6$ represents alkyl or aryl, and $R^{15}$ represents a hydrogen atom or an alkyl, substituted alkyl, aryl, substituted aryl, or cycloalkyl, group;

$R^2$ represents cyano, nitro, carboxy, carboxy ester, carboxyamide, carboxyamide whose amide group is substituted, or a group of formula 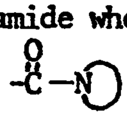 in which 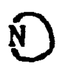 represents a 5-7 ring membered heterocyclic radical which optionally may be substituted;

$R^3$ represents hydrogen, alkyl or a group of formula $-\overset{O}{\overset{\|}{C}}-R^7$ in which $R^7$ represents alkyl, aryl, alkoxy or alkylthio;

$R^4$ represents hydrogen, alkyl or cycloalkyl;

$R^5$ represents hydrogen, alkyl, cycloalkyl, alkoxy, aryl or substituted aryl; or

$R^3$ and $R^5$ together represent alkylene, optionally unsaturated, optionally interrupted by one or more atoms selected from nitrogen, oxygen and sulphur atoms, and optionally substituted.

The invention provides also a process for preparing the compound, which process comprises reacting a 5-aminoisoxazole of formula

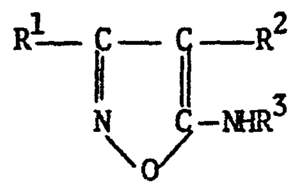

II

with a carbamoyl halide of formula $R^4R^5N\overset{O}{\overset{\|}{C}}Z$, wherein Z represents a halogen atom.

The invention provides also a process for preparing the compound, which process comprises reacting a 5-aminocarboxyisoxazole of formula

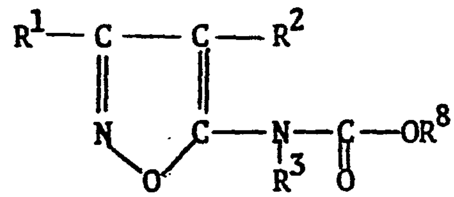

III

wherein $R^8$ represents phenyl or 2,4-dinitrophenyl, with an amine of formula $HNR^4R^5$.

The invention provides also a process for preparing the compound in which $R^3$ represents alkyl or a group of formula $-\overset{O}{\overset{\|}{C}}-R^7$, which process comprises alkylating or acylating the corresponding compound in which $R^3$ represents hydrogen.

The invention also provides a process for preparing the compound in which $R^4$ represents hydrogen, which process comprises reacting the 5-aminoisoxazole of formula II with an isocyanate of formula $R^5NCO$.

The invention also provides a process for preparing the compound, which process comprises reacting a 5-isocyanatoisoxazole of formula

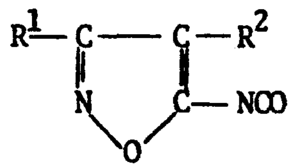

IV

with an amine of formula $HNR^4R^5$.

The invention also provides a process for preparing the compound, which process comprises reacting an isoxazolylcarbamyl halide of formula

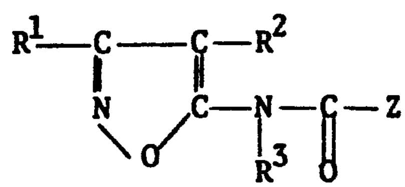

V

with an amine of formula $HNR^4R^5$, where Z represents a halogen atom.

The invention also provides a process for preparing the compound, which process comprises reacting the 5-aminoisoxazole

of formula II with a carbamate of formula $R^8O-\underset{O}{\underset{\|}{C}}-NR^4R^5$ where $R^8$ is as defined above.

The invention also provides a process for preparing the compound, which process comprises reacting a 5-haloisoxazole of formula

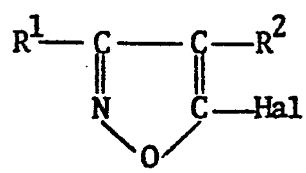

VI

where Hal represents a halogen atom, with a urea of formula

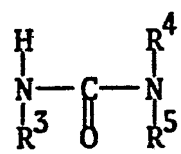

.

The invention also provides a process for preparing the compound in which $R^3$ and $R^5$ together represent alkylene, optionally unsaturated, optionally interrupted by one or more atoms selected from nitrogen, oxygen and sulphur atoms, and optionally substituted, which process comprises reacting the corresponding compound in which $R^3$ and $R^5$ each represent a hydrogen atom with a halide of formula Hal-L-Hal, where L represents $R^3$ and $R^5$ together and Hal represents a halogen atom.

The invention also provides a process for preparing a salt of the 5-isoxazolyl urea, which process comprises salifying the

5-isoxazolyl urea.

The invention also provides a process for preparing the 5-isoxazolyl urea, which process comprises desalifying a salt of the 5-isoxazolyl urea.

The invention also provides a method of combating pests at a locus infested or liable to be infested by them or of regulating the growth of a desired plant at a locus at which the plant is growing or is to grow, which method comprises applying to the locus a pest-combating or plant growth regulant amount of the compound.

The invention provides in addition a pesticidal or plant growth regulant composition containing the compound, especially such a composition comprising the compound together with at least one material selected from carriers, surface active agents, inorganic salts, herbicidal antidotes, fertilizers, other pesticides and other plant growth regulants.

The compound may be prepared by reacting the 5-aminoisoxazole of formula II with the carbamoyl halide of formula $R^4R^5N\overset{O}{\overset{\|}{C}}Z$. Usually Z represents a chlorine atom. Generally the reaction is carried out in an inert solvent and may be carried out at a temperature for example of 0-150°C, e.g. 0-100°C. The reaction is preferably conducted in the presence of an acid acceptor such as an organic base (e.g. pyridine or a tertiary amine such as triethylamine) or, less preferably, an inorganic base (e.g. sodium carbonate or bicarbonate).

The compound may be prepared by reacting the 5-aminocarboxyisoxazole of formula III with an amine of formula $HNR^4R^5$. The reaction may be carried out with or without solvent and may be conducted at a temperature for example of from -10 to 150°C.

The 5-aminocarboxyisoxazole of formula III may itself be prepared by reacting the 5-aminoisoxazole of formula II with a chloroformate of formula $Cl\overset{O}{\overset{\|}{C}}OR^8$. The reaction may be carried out with or without solvent and may be conducted at a temperature for example of from -10 to 150°C.

The present compound in which $R^3$ represents alkyl or a group of formula $-\overset{O}{\overset{\|}{C}}-R^7$ may be prepared by alkylating or acylating the corresponding compound in which $R^3$ represents hydrogen. The reaction may be carried out with or without solvent. Excess reagent may be employed as solvent. The reaction may be conducted at a temperature for example of from -20 to 150°C.

The present compound in which $R^4$ represents hydrogen is preferably prepared by reacting the 5-aminoisoxazole of formula II with an isocyanate of formula $R^5NCO$. The reaction may be carried out in the presence of a catalyst, usually a tertiary amine such as triethylamine. Generally the reaction is carried out in the presence of an inert solvent, for instance a hydrocarbon such as toluene, and may be carried out at a temperature for example of 0-150°C, e.g. 0-120°C.

The present compound may be prepared by reacting the 5-isocyanatoisoxazole of formula IV with the amine of formula $HNR^4R^5$. The reaction is generally carried out in the presence

of an inert solvent, for instance a hydrocarbon such as toluene, and may be carried out at a temperature for example of 0-150°C.

The present compound may be prepared by reacting the isoxazolylcarbamyl halide of formula V with the amine of formula $HNR^4R^5$. Usually Z represents a chlorine atom. Generally the reaction is carried out in an inert solvent and may be carried out at a temperature for example of 0-150°C. The reaction is preferably conducted in the presence of an acid acceptor such as an organic base (e.g. pyridine or a tertiary amine such as triethylamine) or, less preferably, an inorganic base (e.g. sodium carbonate or bicarbonate).

The present compound may be prepared by reacting the 5-aminoisoxazole of formula II with a carbamate of formula $R^8O-\underset{\underset{O}{\|}}{C}-NR^4R^5$. The reaction may be carried out with or without solvent and may be conducted at a temperature for example of from -10 to 150°C.

The present compound may be prepared by reacting the 5-haloisoxazole of formula VI with the urea of formula

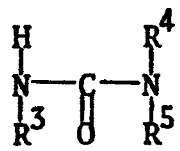 .

Hal preferably represents a bromine atom. The reaction may be carried out with or without solvent and may be carried out at a temperature for example of 0-200°C. The reaction is preferably carried out in the presence of an acid acceptor such as an organic base (e.g. pyridine or a tertiary amine such as triethylamine) or,

less preferably, an inorganic base (e.g. sodium carbonate or bicarbonate).

The present compound in which $R^3$ and $R^5$ together represent a group is preferably prepared by reacting the corresponding compound in which $R^3$ and $R^5$ each represent a hydrogen atom with the halide of formula Hal-L-Hal. Preferably each Hal represents a chlorine atom. The reaction is preferably carried out in an inert solvent and may be carried out for example at a temperature for example of 0-150°C. The reaction may be carried out with or without an acid acceptor. Where an acid acceptor is employed, it may be as discussed above.

The 5-aminoisoxazole of formula II in which $R^3$ represents hydrogen can be prepared by reacting a cyano derivative of formula

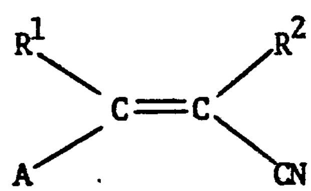

VII

wherein A represents hydroxy, halogen, amino, $-OR^9$, $-SR^9$, $-SOR^9$ or $-SO_2R^9$ where $R^9$ represents alkyl of 1-6 carbon atoms (e.g. methyl or ethyl), with hydroxylamine. The reaction is generally carried out in an inert solvent such as water and may be carried out at a temperature for example of 0-200°C, e.g. 0-100°C. The hydroxylamine can conveniently be formed *in situ* by employing its hydrochloride in the presence of a base such as sodium hydroxide.

The 5-aminoisoxazole of formula II wherein $R^1$ is linked to the heterocycle via a carbon atom in $R^1$ may be prepared by reacting a salt of formula $M^{\oplus}\underset{CN}{\overset{\ominus}{C}}HR^{2}$ where $M^{\oplus}$ represents a cation with a nitrile oxide of formula $R^{1}\overset{\oplus}{C}{=}N{-}\overset{\ominus}{O}$ or with the corresponding hydroxamyl chloride of formula $R^{1}\overset{Cl}{C}{=}NOH$. The nitrile oxide may be prepared from the hydroxamyl chloride. The nitrile oxide may be formed <u>in situ</u> and reacted immediately with the salt. The present reaction is generally conducted in an inert solvent and may be carried out for example at a temperature of from -100 to 150°C.

The 5-aminoisoxazole of formula II wherein $R^1$ represents hydrogen, alkyl, substituted alkyl, cycloalkyl, aryl, substituted aryl or amino may be prepared by reacting the 5-haloisoxazole of formula VI having this value of $R^1$ with ammonia. The reaction may be carried out with or without an inert solvent and may be carried out at a temperature for example of 0-200°C. The reaction may be conducted in the presence of an acid acceptor, which may be as discussed above.

The 5-haliosoxazole of formula VI may be prepared by halogenating with a halogenating agent (e.g. thionyl chloride) the corresponding 5-hydroxyisoxazole. The reaction may be carried out with or without an inert solvent and may be carried out for example at a temperature of 0-100°C. The reaction may be conducted in the presence of a catalyst such as dimethylformamide.

The 5-hydroxyisoxazole may be prepared by reacting the unsaturated derivative of formula

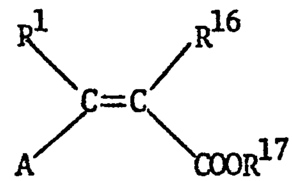

VIII

where $R^{16}$ represents $R^2$ except cyano (e.g. ethyl) and $R^{17}$ represents alkyl of 1-4 carbon atoms (e.g. ethyl) with hydroxylamine. $R^1$ for instance may represent ethyl and A hydroxy. The reaction is generally carried out in an inert solvent such as water and may be carried out at a temperature for example of 0-200°C. The hydroxylamine can conveniently be formed in situ by employing its hydrochloride in the presence of a base such as sodium hydroxide.

Some of the present intermediates are novel, and the invention provides them and their production. In particular, there are provided as novel compounds:

Ethyl 5-amino-3-ethylisoxazole-4-carboxylate,
propyl 5-amino-3-methylisoxazole-4-carboxylate,
isopropyl 5-amino-3-methylisoxazole-4-carboxylate,
butyl 5-amino-3-methylisoxazole-4-carboxylate,
propyl 5-amino-3-ethylisoxazole-4-carboxylate,
isopropyl 5-amino-3-ethylisoxazole-4-carboxylate, and
ethyl 5-amino-3-propylisoxazole-4-carboxylate, especially the first of these.

When $R^1$ represents a substituted amino group, the amino group may be mono- or di-substituted. Any substituent may be for example alkyl or alkylcarbonyl. The substituted amino group may

be for instance dimethylamino or acetamido.

When $R^1$ represents a substituted alkyl, aryl or aralkyl group, or $R^{15}$ represents a substituted alkyl or aryl group, the substituent(s) may be for example one or more substituents selected from halogen, alkoxy, nitro and, except when $R^1$ or $R^{15}$ represents substituted alkyl, alkyl.

When $R^1$ represents a substituted aralkyl group, the substituent(s) are usually on the aryl part of the group.

When $R^2$ represents carboxyamide whose amide group is substituted, the amide group may be mono- or di-substituted. Any substituent may be for example alkyl or alkylcarbonyl.

The group of formula 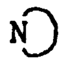 which $R^2$ may contain is generally unsaturated. It may be for instance a 5-7 ring membered heterocyclic group containing in the ring besides the nitrogen atom either none or one other hetero atom selected from nitrogen and oxygen. It may be for example pyrrol-1-yl, morpholino, piperidino or hexamethyleneimino. The group may be substituted, e.g. by alkyl; the group may be for instance 2,6-dimethylmorpholino.

The alkylene group which $R^3$ and $R^5$ may together represent is usually of 1-5 carbon atoms. It may be substituted, e.g. by one or more groups selected from halogen, hydroxy, alkoxy, oxo and alkanoyloxy groups.

When $R^5$ represents a substituted aryl group, the substituent may be for example one or more substituents selected from halogen, alkoxy, alkyl and nitro.

When any of the present symbols represents a substituted or unsubstituted group, it is preferably unsubstituted. When it is substituted, the substituents are usually the same, e.g. all chlorine or all methyl, and preferably any such substituted group is monosubstituted.

Any alkyl group involved in formula I is preferably of 1-10, e.g. 1-6, carbon atoms, for example methyl, ethyl, propyl, isopropyl or butyl. Any aryl group is preferably phenyl. Any aralkyl group is preferably phenylalkyl of 7-10 carbon atoms, especially benzyl. Any halogen (i.e. fluorine, chlorine, bromine or iodine) is preferably fluorine, chlorine or bromine especially chlorine. Any cycloalkyl group is preferably of 3-7 carbon atoms especially cyclohexyl. Any alkoxy or alkylthio group is preferably of 1-6 carbon atoms e.g. methoxy or methylthio. Any alkanoyloxy group is preferably of 2-7 carbon atoms.

The carboxyester group which $R^2$ may represent is preferably alkoxycarbonyl e.g. of 2-7 carbon atoms, (for instance methoxycarbonyl), phenoxycarbonyl whose phenyl group may be substituted (e.g. by one or more substituents selected from halogen, alkoxy, alkyl and nitro), or aralkyloxycarbonyl (e.g. benzyloxycarbonyl).

Thus, in a preferred embodiment, the compound is of formula I or a salt thereof, wherein $R^1$ represents a hydrogen or halogen atom; nitro; amino; amino substituted by one or two substituents selected from alkyl of 1-10 carbon atoms and alkylcarbonyl of 2-7 carbon atoms; alkyl of 1-10 carbon atoms; alkyl of 1-10 carbon

atoms substituted by one or more substituents selected from halogen, alkoxy of 1-6 carbon atoms and nitro; cycloalkyl of 3-7 carbon atoms; phenyl; phenyl substituted by one or more substituents selected from halogen, alkoxy of 1-6 carbon atoms, nitro and alkyl of 1-10 carbon atoms; phenylalkyl of 7-10 carbon atoms; phenylalkyl of 7-10 carbon atoms whose phenyl group is substituted by one or more substituents selected from halogen, alkoxy of 1-6 carbon atoms, nitro and alkyl of 1-10 carbon atoms; or a group of formula $-XR^6$ or $COOR^{15}$ in which X represents oxygen, sulphur, sulphinyl or sulphonyl, $R^6$ represents alkyl of 1-10 carbon atoms or phenyl, and $R^{15}$ represents a hydrogen atom; alkyl of 1-10 carbon atoms; alkyl of 1-10 carbon atoms substituted by one or more substituents selected from halogen, alkoxy of 1-6 carbon atoms and nitro; phenyl; phenyl substituted by one or more substituents selected from halogen, alkoxy of 1-6 carbon atoms, nitro and alkyl of 1-10 carbon atoms; or cycloalkyl of 3-7 carbon atoms;

$R^2$ represents cyano; nitro; carboxy; alkoxycarbonyl of 2-7 carbon atoms; phenoxycarbonyl whose phenyl group optionally may be substituted by one or more substituents selected from halogen, alkoxy of 1-6 carbon atoms, alkyl of 1-10 carbon atoms and nitro; benzyloxycarbonyl; carboxyamide; carboxyamide whose amide group is substituted by one or two substituents selected from alkyl of 1-10 carbon atoms and alkylcarbonyl of 2-7 carbon atoms; or a group of formula 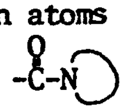 in which 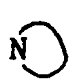 represents a 5-7 ring membered heterocyclic group containing in the ring besides the nitrogen

atom either none or one other hetero atom selected from nitrogen and oxygen, which group optionally may be substituted by alkyl of 1-10 carbon atoms;

$R^3$ represents hydrogen, alkyl of 1-10 carbon atoms or a group of formula $-\overset{O}{\overset{\|}{C}}-R^7$ in which $R^7$ represents alkyl of 1-10 carbon atoms, phenyl, alkoxy of 1-6 carbon atoms or alkylthio of 1-6 carbon atoms;

$R^4$ represents hydrogen, alkyl of 1-10 carbon atoms or cycloalkyl of 3-7 carbon atoms;

$R^5$ represents hydrogen, alkyl of 1-10 carbon atoms, cycloalkyl of 3-7 carbon atoms, alkoxy of 1-6 carbon atoms, phenyl, or phenyl substituted by one or more substituents selected from halogen, alkoxy of 1-6 carbon atoms, nitro and alkyl of 1-10 carbon atoms; or

$R^3$ and $R^5$ together represent an alkylene group of 1-5 carbon atoms, which group is optionally unsaturated, optionally interrupted by one or more atoms selected from nitrogen, oxygen and sulphur atoms, and optionally substituted by one or more groups selected from halogen, hydroxy, alkoxy of 1-6 carbon atoms, oxo and alkanoyloxy of 2-7 carbon atoms.

The present compounds may form salts with acids or bases. These may be formed from the non-salt form (i.e. the non-salt form may be salified) in conventional ways e.g. by reaction of the non-salt form with an acid or base. The non-salt form may be formed from the salt (i.e. the salt may be desalified) in conventional ways; a salt with an acid may be reacted with a

base, or a salt with a base may be reacted with an acid. Salts may be formed from a compound in which $R^1$ or $R^2$ represents carboxy, e.g. by reaction of the carboxy compound with a base for example an alkali metal hydroxide or an amine. Salts include metal cation, e.g. sodium or potassium, salts, and amine (e.g. methylamine) or substituted amine (e.g. ethanolamine), salts. The compounds in which $R^1$ or $R^2$ represents carboxy may be formed from the salts by reaction with acids e.g. hydrochloric acid to form the carboxy compounds. Salts may be formed from a compound in which $R^1$ represents amino or substituted amino, e.g. by reaction of the compound with an acid e.g. hydrochloric acid. Salts may also be formed from a compound in which $R^3$, $R^4$ or $R^5$ represents a hydrogen atom; thus salts with bases, e.g. alkali metal salts, may be formed.

The reaction to form salts from the non-salt form, or the non-salt form from salts, is usually carried out at a temperature of 0-150$^oC$, generally in the presence of an inert solvent.

In a preferred embodiment, the compound is of formula I in which

$R^1$ represents alkyl of 1-4 carbon atoms, $R^2$ represents cyano, nitro or alkoxycarbonyl of 2-5 carbon atoms, $R^3$ represents hydrogen, $R^4$ represents hydrogen, alkyl of 1-4 carbon atoms or cyclohexyl, $R^5$ represents alkyl of 1-4 carbon atoms, cyclohexyl, 4-chlorophenyl or 3-chlorophenyl, or $R^3$ and $R^5$ together represent 2,4,5-trioxoimidazolidin-1-yl.

In a narrower group within this embodiment, $R^1$ represents alkyl of 1-4 carbon atoms, $R^2$ represents cyano, nitro or alkoxycarbonyl of 2-5 carbon atoms, $R^3$ and $R^4$ each represents hydrogen, and $R^5$ represents alkyl of 1-4 carbon atoms, cyclohexyl, 4-chlorophenyl or 3-chlorophenyl.

Particular formula I compounds are specified in the Examples.

Particular preferred compounds are
ethyl 3-methyl-5-(3-ethylureido)isoxazole-4-carboxylate,
propyl 3-methyl-5-(3-ethylureido)isoxazole-4-carboxylate,
ethyl 3-methyl-5-(3-methylureido)isoxazole-4-carboxylate,
propyl 3-ethyl-5-(3-ethylureido)isoxazole-4-carboxylate,
propyl 3-ethyl-5-(3-butylureido)isoxozole-4-carboxylate,
butyl 3-methyl-5-(3-ethylureido)isoxazole-4-carboxylate,
ethyl 3-ethyl-5-(3-tert butylureido)isoxazole-4-carboxylate,
propyl 3-ethyl-5-(3-isopropylureido)isoxazole-4-carboxylate,
propyl 3-ethyl-5-(3-propylureido)isoxazole-4-carboxylate,
propyl 3-methyl-5-(3-butylureido)isoxazole-4-carboxylate,
ethyl 3-ethyl-5-(3-ethylureido)isoxazole-4-carboxylate,
ethyl 3-ethyl-5-(3-propylureido)isoxazole-4-carboxylate,
ethyl 3-ethyl-5-(3-butylureido)isoxazole-4-carboxylate, and
ethyl 3-ethyl-5-(3-isopropylureido)isoxazole-4-carboxylate,
especially the last seven, particularly the last three.

The present compounds are pesticides and plant growth regulants. The compounds are outstandingly active on plant physiology, affecting the growth of plants so that the compounds may be used as herbicides

or plant growth regulants, especially as selective herbicides. They are also fungicides. They are particularly useful for controlling weeds in crops.

For use as fungicides, the compounds are preferably employed to combat fungal diseases of plants.

The compounds are normally employed in the form of compositions, which can be prepared by admixing the ingredients. Usually the compositions are initially produced in the form of concentrates, e.g. containing 0.5-85% of the present compound, and these are diluted with water or hydrocarbon, usually water, for application, generally such that the concentration of the compound is 0.05-5%, though in ultra low volume application the concentration may be higher, e.g. up to 20%. Percentages, proportions and parts in this specification are by weight unless otherwise indicated.

The compositions normally contain a surface active agent and/or a carrier.

The carrier may be a liquid, e.g. water (e.g. water used to dilute a concentrate for application). If water is employed as carrier in a concentrate, an organic solvent may also be present as carrier, though this is not usually employed. A surface active agent may advantageously be present.

The carrier may be a liquid other than water, for example an organic solvent, such as a water immiscible solvent, e.g. a hydrocarbon which boils within the range 130-270°C, in which the compound is dissolved or suspended. A concentrate containing a

water immiscible solvent suitably also contains a surface active agent so that the concentrate acts as a self-emulsifiable oil on admixture with water.

The carrier may be a solid, which may be finely divided. Examples of suitable solids are limestone, clays, sand, mica, chalk, attapulgite, diatomite, perlite, sepiolite, silicas, silicates, lignosulphonates, peat and solid fertilizers. The carrier can be of natural or synthetic origin or can be a modified natural material.

Wettable powders soluble or dispersable in water may be formed by admixing the compound in particulate form with a particulate carrier or spraying molten compound on to the particulate carrier, admixing a wetting agent and a dispersing agent and finely grinding the whole powder mixture.

An aerosol composition may be formed by admixing the compound with a propellant e.g. a polyhalogenated alkane such as dichlorodifluoromethane, and suitably also with a solvent.

A flowable suspension concentrate may be formed by grinding the compound with water, a wetting agent and a suspending agent.

A flowable suspension concentrate wherein the carrier is a hydrocarbon which boils within the range 130-270$^{o}$C rather than water may be formed.

Thus the present composition can for example be solid (e.g. dust or granules) and contain a solid carrier, or liquid (e.g. an emulsifiable concentrate) and contain a liquid carrier which is a

hydrocarbon which boils within the range 130-270$^{o}$C.

The term 'surface active agent' is used in the broad sense to include materials variously called emulsifying agents, dispersing agents and wetting agents. Such agents are well known in the art.

The surface active agents used may comprise anionic surface active agents, for example mono- or di-esters of phosphoric acid with fatty alcohol ethoxylates or salts of such esters, fatty alcohol sulphates such as sodium dodecyl sulphate, ethoxylated fatty alcohol sulphates, ethoxylated alkylphenol sulphates, lignin sulphonates, petroleum sulphonates, alkyl-aryl sulphonates such as alkyl-benzene sulphonates or lower alkyl-naphthalene sulphonates, salts of sulphonated naphthalene-formaldehyde condensates, salts of sulphonated phenol-formaldehyde condensates, or more complex sulphonates such as the amide sulphonates e.g. the sulphonated condensation product of oleic acid and N-methyl taurine or the dialkyl sulphosuccinates e.g. the sodium sulphonate of dioctyl succinate.

The surface active agents may also comprise non-ionic agents, for example condensation products of fatty acid esters, fatty alcohols, fatty acid amides or alkyl-substituted phenols with ethylene oxide, fatty esters of polyhydric alcohol ethers e.g. sorbitan fatty acid esters, condensation products of such esters with ethylene oxide e.g. polyoxyethylene sorbitan fatty acid esters, block copolymers of ethylene oxide and propylene oxide, acetylenic glycols such as 2,4,7,9-tetramethyl-5-decyn-4,7-diol,

or ethoxylated acetylenic glycols.

The surface active agents may also comprise cationic agents, for example alkyl- and/or aryl-substituted quaternary ammonium compounds such as cetyl trimethylammonium bromide, or ethoxylated tertiary fatty amines.

Preferred surface active agents include ethoxylated fatty alcohol sulphates, lignin sulphonates, alkyl-aryl sulphonates, salts of sulphonated naphthalene-formaldehyde condensates, salts of sulphonated phenol-formaldehyde condensates, sodium oleoyl N-methyltauride, dialkyl sulphosuccinates, alkyl phenol ethoxylates, and fatty alkyl ethoxylates.

Higher quantities of surface active agent, e.g. 5-50% of a concentrate, than is normally present in commercial pesticidal or plant growth regulant compositions may be employed to increase the activity of the compounds.

The present active compound may be admixed with another pesticide, e.g. herbicide, fungicide or insecticide, or with another plant growth regulant. The invention provides a one pack presentation, in which the present compound is already mixed with other pesticide or plant growth regulant, and also a single package designed to hold the present compound and other pesticide or plant growth regulant in separate containers, for mixing, e.g. in a spray tank, for application. Particular advantages are obtained with mixtures with another herbicide. The present compound may be used sequentially with another pesticide or plant

growth regulant particularly with another herbicide, e.g. one herbicide applied before planting or before emergence of a crop and the other herbicide applied after emergence of the crop.

The other herbicide may be for example one or more of a phenoxyaliphatic acid, substituted urea, triazine, phenol, nitrile, bipyridylium compound, substituted benzoic acid, halogenated aliphatic acid, carbamate, thiocarbamate, chloroacetamide, diazine or arsenic herbicide. In respect of selective herbicidal compositions for post-emergence use, the present compound may be used in admixture with for example a substituted phenoxyaliphatic acid; in respect of selective herbicidal compositions for pre-emergence use, the present compound may be used in admixture with for example a substituted urea or triazine; in respect of sequential selective herbicidal use, one may apply for example before emergence of the crop S-2,3-dichloroallyl di-isopropylthiocarbamate or S-2,3,3-trichloroallyl di-isopropylthiocarbamate and the present compound after emergence of the crop.

The phenoxyaliphatic acid generally comprises alkyl and/or halogen substituted phenoxyaliphatic acids, and their salts, for example alkali metal, amine and alkanolamine salts, and functional derivatives, for example esters and amides. These compounds may be of activity such that they are recognised as commercial herbicides, or may be of only slight herbicidal activity. Examples of the substituted phenoxyaliphatic acids which may be mentioned include 2,4-dichlorophenoxyacetic acid, 2-(2,4-dichlorophenoxy)-propionic acid, 2-methyl-4-chlorophenoxyacetic acid, 2,4,5-trichlorophenoxyacetic acid, gamma-2,4-dichlorophenoxybutyric acid, gamma-2-methyl-4-chlorophenoxybutyric acid, alpha-2-methyl-4-chlorophenoxypropionic acid, 2-(4-[2,4-dichlorophenoxy]phenoxy) propionic acid and 2-(4-[4-chlorophenoxy]phenoxy)propionic acid.

The substituted urea generally comprises a tri- or tetra-substituted urea such as N'-(3-chloro-4-methoxyphenyl)-N,N-dimethylurea, N'-(3-chloro-4-methylphenyl)-N,N-dimethylurea, N-parachlorophenyl-N,N-dimethylurea, N-butyl-N'-(3,4-dichlorophenyl)-N-methylurea, N'-parachlorophenyl-O,N,N-trimethylisourea, N'-p-chlorophenyl-N-methoxy-N-methylurea, N,N-dimethyl-N'-phenylurea, 3-(4-bromophenyl)-1-methoxy-1-methylurea, 1-(2-benzothiazolyl)-3-methylurea, N,N-dimethyl-N'-(4-[1-methylethyl]phenyl)urea, N'-(3,4-dichlorophenyl)-N-methoxy-N-methylurea or N,N-dimethyl-N'-[3-(trifluoromethyl)phenyl]urea.

The triazine herbicide generally comprises 2-chloro-4-(1-cyano-1-methylamino)-6-ethylamino-1,3,5-triazine or 2-isopropylamino-4-(3-methoxypropylamino)-6-methylthio-1,3,5-triazine or a compound of the formula:-

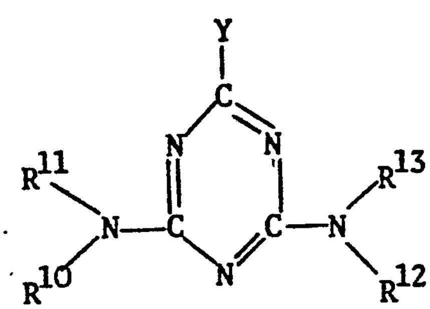

where Y is a halogen atom, OR group or SR group, where R is an alkyl group, $R^{11}$ and $R^{13}$ are the same or different and are hydrogen or alkyl and $R^{10}$ and $R^{12}$ are the same or different alkyl groups, such as 2-chloro-4,6-bisethylamino-1,3,5-triazine, 2-chloro-6-ethylamino-4-isopropylamino-1,3,5-triazine, or 2,4-bis(isopropylamino)-6-methylthio-1,3,5-triazine.

The phenol herbicide generally comprises 4,6-dinitro-o-cresol,4,6-dinitro-2-sec-butylphenol or pentachlorophenol. The nitrile herbicide generally comprises 3,5-diiodo-4-hydroxybenzonitrile, 3,5-dibromo-4-hydroxybenzonitrile or 2,6-dichlorobenzonitrile. The bipyridylium herbicide generally comprises 1,1'-dimethyl-4,4'-bipyridylium dichloride or 1,1'-ethylene-2,2'-bipyridylium dibromide. The substituted benzoic acid herbicide generally comprises 2,3,6-trichlorobenzoic acid, 2-methoxy-3,6-dichlorobenzoic acid or N-(1,1-dimethylpropynyl)-3,5-dichlorobenzamide. The halogenated aliphatic acid herbicide generally comprises trichloroacetic acid or 2,2-dichloropropionic acid. The carbamate herbicide generally comprises isopropyl N-(3-chlorophenyl) carbamate, 4-chloro-2-butynyl N-(3-chlorophenyl)carbamate, methyl 3-(m-tolylcarbamoyloxy)phenylcarbamate or D-N-ethyl-2-(phenylcarbamoyloxy)propionamide. The thiocarbamate herbicide generally comprises S-ethyl N,N-dipropylthiocarbamate, S-ethyl N,N-diisobutylthiocarbamate, S-(2,3-dichloroallyl) N,N-diisopropylthiocarbamate, S-ethyl N-ethyl-N-cyclohexylthiocarbamate, S-propyl butylethylthiocarbamate or S-(2,3,3-trichloroallyl) N,N-diisopropyl-

thiocarbamate. The chloroacetamide herbicide generally comprises N,N-diallyl-2-chloroacetamide or N-isopropyl-2-chloroacetanilide. The diazine herbicide generally comprises 5-bromo-6-methyl-3-sec-butyluracil, 3-cyclohexyl-5,6-trimethyleneuracil, 5-amino-4-chloro-2-phenyl-3-pyridazinone or 1,2-dihydropyridazine-3,6-dione. The arsenic herbicide generally comprises a salt of methane arsonic acid or cacodylic acid. Other herbicides which may be used as the second herbicide include 1,2-dimethyl-3,5-diphenyl-pyrazolium ion, ethyl N-benzoyl-N-(3,4-dichlorophenyl)alanine, N-isobutyl-2-oxo-1-imidazolidine-carboxamide, aminotriazole,2,3-dichloro-1,4-naphthoquinone, 4-amino-3,5,6-trichloropicolinic acid, N,N-dimethyl-2,2-diphenylacetamide, 2,6-dinitro-N,N-dipropyl-4-trifluoromethylaniline, N-butyl-N-ethyl-2,6-dinitro-4-trifluoromethylaniline, S,S,S-tributyl phosphorotrithioate, 2-ethoxy-2,3-dihydro-3,3-dimethyl-5-benzofuranyl methylsulphonate, 4-chloro-2-oxobenzothiazolin-3-yl acetic acid, 3-isopropyl-2,1,3-benzothiadiazinon-(4)-2,2-dioxide, 3,5-dibromo-4-hydroxybenzaldehyde 2,4-dinitrophenyloxime, methyl 2-chloro-3-(4-chlorophenyl) propionate, 2-chloroethyltrimethylammonium chloride, 4-methylsulphonyloxy-2-butynyl m-chlorocarbanilate, isopropyl 2-(N-benzoyl-3-chloro-4-fluoroanilino)propionate, methyl 2-(N-benzoyl-3-chloro-4-fluoroanilino)propionate, 2-chloro-N-(1,3-dioxolan-2-ylmethyl)-2',6'-dimethylacetanilide, 2-chloro-1-(3-ethoxy-4-nitrophenoxy)-4-trifluoromethylbenzene, methyl 2-(4-[2',4'-dichlorophenoxy] phenoxy)propionate or isobutyl 2-(4-[4'-chlorophenoxy]phenoxy)

propionate.

The other herbicide may particularly be another cereal herbicide, e.g. one which combats monocotyledonous weeds or cleavers in a cereal crop.

The present compound may be used in admixture or sequence with a fungicide, particularly a cereal fungicide. The fungicide may be for instance one or more of maneb (polymeric manganese ethylenebisdithiocarbamate), zineb (zinc ethylenebisdithiocarbamate), mancozeb (which can be regarded as a mixture of maneb and zineb), thiram (tetramethylthiuram disulphide), ditalimfos (O,O-diethyl phthalimidophosphonothioate), tridemorph (2,6-dimethyl-4-tridecylmorpholine), fluotrimazole (1-[diphenyl-(3-trifluoromethylphenyl)methyl]-1,2,4-triazole), ethirimol (5-butyl-2-ethylamino-4-hydroxy-6-methylpyrimidine), triforine (1,4-di[2,2,2-trichloro-1-formamidoethyl]piperazine), pyracarbolid (3,4-dihydro-6-methylpyran-5-carboxanilide), zinebethylene thiuramdisulphide adduct, carbendazim (methyl benzimidazol-2-ylcarbamate), captafol (3a,4,7,7a-tetrahydro-N-[1,1,2,2-tetrachloroethanesulphenyl]-phthalimide), thiophanate (1,2-di[3-ethoxycarbonyl-2-thioureido]-benzene), propineb (polymeric zinc propylenebisdithiocarbamate), oxycarboxin (2,3-dihydro-6-methyl-5-phenylcarbamoyl-1,4-oxathiin 4,4-dioxide), quintozene (pentachloronitrobenzene), benomyl (methyl 1-[butylcarbamoyl] benzimidazol-2-ylcarbamate), benadanil (2-iodobenzanilide), dichlofluanid (N-dichlorofluoromethane-sulphenyl-N',N'-dimethyl-N-phenylsulphamide), sulphur, copper

compounds, iprodione (3-/̲3,5-dichlorophenyl̲7-1-/̲(1-methylethyl)-aminocarbonyl̲7-imidazolidine-2,4-dione, ziram (zinc dimethyldithiocarbamate), nabam (disodium ethylenebisdithiocarbamate), and triadimefon (1-(4-chlorophenoxy)-3,3-dimethyl-1-(1,2,4-triazol-1-yl)-2-butanone).

The present compound may be used in admixture or sequence with an insecticide, particularly a cereal insecticide. The insecticide may be for instance one or more of demeton-S-methyl (S-2-ethylthioethyl O,O-dimethyl phosphorothioate), dimethoate (O,O-dimethyl S-methylcarbamoylmethyl phosphorodithioate), formothion (S-/̲N-formyl-N-methylcarbamoylmethyl̲7 O,O-dimethyl phosphorodithioate), oxydemeton-methyl (S-2-ethylsulphinylethyl O,O-dimethyl phosphorothioate), pirimicarb (2-dimethylamino-5,6-dimethylpyrimidin-4-yl dimethylcarbamate), thiometon (S-2-ethylthioethyl O,O-dimethyl phosphorodithioate), BHC (benzene hexachloride), aldrin (1,2,3,4,10,10-hexachloro-1,4a,5,8,8a-hexahydro-exo-1,4-endo-5,8-dimethanonaphthalene), fenitrothion (O,O-dimethyl O-4-nitro-<u>m</u>-tolyl phosphorothioate), omethoate (O,O-dimethyl S-methylcarbamoylmethyl phosphorothioate), pirimiphos-methyl (O-2-diethylamino-6-methyl-pyrimidin-4-yl O,O-dimethyl phosphorothioate) and DDT (1,1,1-trichloro-2,2-di/̲chlorophenyl̲7ethane).

The ratio of the present compound to the other pesticide or plant growth regulant may vary over a wide range according to the particular compounds involved and the intended use. In general the ratio of present compound to other pesticide or plant growth

regulant lies in the range 1:0.1 to 1:15.

In a particular embodiment, ethyl 3-ethyl-5-(3-propylureido)-isoxazole-4-carboxylate is employed as a selective herbicide in combination with chlortoluron (N'-(3-chloro-4-methylphenyl)-N,N-dimethylurea) and optionally mecoprop (2-(4-chloro-2-methylphenoxy)-propanoic acid or a salt thereof), e.g. at rates respectively of 0.3, 2.5 and 2.0, kg per hectare.

The present compounds may be in admixture with non-phytotoxic oils, e.g. Fyzol 11E, Agri-Oil Plus or Sun Oil 11E.

The compounds may be employed in association with a herbicidal antidote (a substance having the property of improving the safety to crops of the compounds), e.g. N,N-diallyl-2,2-dichloro-acetamide, 4'-chloro-2-hydroxyiminoacetanilide or 1,8-naphthalic anhydride. Although the antidote may be applied in admixture with one or more of the essential compounds, it is preferably applied separately, and especially as a treatment for crop seeds. The ratio by weight of total herbicide to antidote is preferably from 1:2 to 8:1.

The compounds may be in admixture with an inorganic salt such as ammonium sulphate which improves the activity of some herbicides. For this, it may be applied for example at 0.5-20, e.g. 2, kg per hectare, and may be admixed with the present compound in a spray tank immediately before use.

The compounds may be in admixture with fertilizers.

In the use of the present compounds as total herbicides, high

rates of application, for example at least 10 kg per hectare, such as 10-25 kg per hectare, of the compounds are usually required, unless they are mixed with other herbicides, in which case the rate can be reduced.

In the use of the present compounds as selective herbicides, the rate of application is usually much lower and may be for example 0.05-10, preferably 0.1-4, kg per hectare. In a particular embodiment, the rate as a selective herbicide is 0.5-10, e.g. 0.5-8, kg per hectare, such as 1-4 kg per hectare.

In the use of the compounds as plant growth regulants, low rates of application are usually required such as 0.1-4, e.g. 0.5-1, kg per hectare.

For use as fungicides, the compounds are generally applied at a rate of 1-6 kg per hectare.

The present compounds may be applied to plants, the soil, land or aquatic areas, or to inanimate or stored materials, e.g. textiles, paper, leather or wood, susceptible to fungal attack. They are outstanding herbicides, especially for selectively combating weeds by application to a locus at which a crop is growing or is to grow. Thus, the compounds may be applied pre- or post-planting of the crop. They may be employed for pre-emergence use or post-emergence use. The crop may be for instance a cereal crop, e.g. maize (Zea mais), wheat (Triticum aestivium), barley (Hordeum vulgare), rye (Lolium spp.), oats (Avena sativa) or rice (Oryza sativa), peas (Pisum sativum), ryegrass (Lolium perenne), potatoes (Solanum spp),

onions (Allium cepa), navy beans (Phaseolus vulgaris), sunflowers (Helianthus spp), timothy grass,(Phleum spp), peanuts (Arachis hypogea), soyabeans (Glycine max) or cotton (Gossypium spp), especially a cereal crop or potatoes, particularly a winter cereal crop.

The present compounds are active against a wide range of weeds, e.g. monocotyledonous weeds such as wild oats or blackgrass, and particularly active against broad-leaved weeds.

They are active for example against wild oats (Avena fatua), blackgrass (Alopecurus myosuroides), fat hen (Chenopodium album), purslane (Portulaca oleracea), prickly sida (Sida spinoso), chickweed (Stellaria media), mayweed (Tripleurospermum maritimum), pale persicarea (Polygonum lapathifolium), speedwell (Veronica persica), pigweed (Amaranthus retroflexus), mustard (Sinapis alba), annual morning glory (Ipomoea annua) and sicklepod (Cassia obtusifolia).

The present compounds are also fungicides. They are active against fungal diseases of plants, e.g. rust fungal diseases. For use as fungicides, the compounds are preferably applied to crops, especially cereals or beans.

Thus, the compounds can be used to protect plants from weeds and fungus.

The invention is illustrated by the following Examples.

Example 1

Ethyl 2-cyano-3-ethoxy-2-pentenoate (105 parts) was slowly added to a stirred solution of hydroxylamine hydrochloride (48 parts) and sodium hydroxide (27 parts) in water (250 parts). The mixture was stirred for four hours, maintaining the temperature below $50^{o}$. The crystalline solid was then filtered off and recrystallised from ethanol to give ethyl 5-amino-3-ethylisoxazole-4-carboxylate (42 parts), melting point 128-130°C.

Analysis: Found: C, 51.99; H, 6.71; N, 14.91%

$C_8H_{12}N_2O_3$ requires: C, 52.16; H, 6.57; N, 15.21%

A mixture of this compound (40 parts) and ethyl isocyanate (45 parts) in a solution of triethylamine (4 parts) in toluene (350 parts) was heated under reflux for 18 hours. The mixture was evaporated to dryness and the residue recrystallised from cyclohexane to give ethyl 3-ethyl-5-(3-ethylureido)isoxazole-4-carboxylate (32 parts), melting point 65-67°C.

Analysis: Found: C, 52.08; H, 6.94; N, 16.23%

$C_{11}H_{17}N_3O_4$ requires: C, 51.75; H, 6.71; N, 16.46%

Examples 2-8

The following compounds were prepared by methods analogous to that of the first part of Example 1:

Propyl 5-amino-3-methylisoxazole-4-carboxylate m.p. = 134-136°

Isopropyl 5-amino-3-methylisoxazole-4-carboxylate m.p. = 120-122°

Butyl 5-amino-3-methylisoxazole-4-carboxylate m.p. = 100-102°

Ethyl 5-amino-3-ethylisoxazole-4-carboxylate m.p. = 128-130°

Propyl 5-amino-3-ethylisoxazole-4-carboxylate m.p. = 125-127°

Isopropyl 5-amino-3-ethylisoxazole-4-carboxylate m.p. = 102-105°

Ethyl 5-amino-3-propylisoxazole-4-carboxylate m.p. = 108-110°

Examples 9-53

The following compounds of formula I in which $R^3$ and $R^4$ represent hydrogen and $R^2$ represents a group of formula $-COOR^{14}$ were prepared by methods analogous to that described in Example 1. All gave satisfactory elemental analysis.

| Example No | $R^1$ | $R^{14}$ | $R^5$ | m.p. (°C) |
|---|---|---|---|---|
| 9 | $CH_3$ | $CH_3$ | $\underline{n}-C_4H_9$ | 112-114 |
| 10 | $CH_3$ | $C_2H_5$ | $CH_3$ | 156-158 |
| 11 | $CH_3$ | $C_2H_5$ | $C_2H_5$ | 112-114 |
| 12 | $CH_3$ | $C_2H_5$ | $\underline{n}-C_3H_7$ | 134-135 |
| 13 | $CH_3$ | $C_2H_5$ | $\underline{iso}-C_3H_7$ | 134-135 |
| 14 | $CH_3$ | $C_2H_5$ | $\underline{n}-C_4H_9$ | 105-107 |
| 15 | $CH_3$ | $C_2H_5$ | cyclohexyl | 147-148 |
| 16 | $CH_3$ | $\underline{n}-C_3H_7$ | $C_2H_5$ | 108-110 |
| 17 | $CH_3$ | $\underline{n}-C_3H_7$ | $\underline{n}-C_4H_9$ | 76-78 |
| 18 | $CH_3$ | $\underline{iso}-C_3H_7$ | $CH_3$ | 91-94 |
| 19 | $CH_3$ | $\underline{iso}-C_3H_7$ | $C_2H_5$ | 89-91 |
| 20 | $CH_3$ | $\underline{iso}-C_3H_7$ | $\underline{n}-C_3H_7$ | 79-81 |
| 21 | $CH_3$ | $\underline{iso}-C_3H_7$ | $\underline{iso}-C_3H_7$ | 78-81 |
| 22 | $CH_3$ | $\underline{iso}-C_3H_7$ | $\underline{n}-C_4H_9$ | 105-106 |
| 23 | $CH_3$ | $\underline{iso}-C_3H_7$ | $4-ClC_6H_4$ | 172-173 |

| Example No | $R^1$ | $R^{14}$ | $R^5$ | m.p. (°C) |
|---|---|---|---|---|
| 24 | $C_2H_5$ | $C_2H_5$ | iso-$C_3H_7$ | 122-124 |
| 25 | $C_2H_5$ | $C_2H_5$ | n-$C_3H_7$ | 64-66 |
| 26 | $C_2H_5$ | $C_2H_5$ | n-$C_4H_9$ | 81-83 |
| 27 | $C_2H_5$ | $C_2H_5$ | cyclohexyl | 112-114 |
| 28 | $C_2H_5$ | n-$C_3H_7$ | $C_2H_5$ | 88-90 |
| 29 | $C_2H_5$ | $CH_3$ | $C_2H_5$ | 127-129 |
| 30 | $C_2H_5$ | $CH_3$ | n-$C_3H_7$ | 132-134 |
| 31 | $C_2H_5$ | $CH_3$ | iso-$C_3H_7$ | 130-131 |
| 32 | $CH_3$ | $CH_3$ | $C_2H_5$ | 169-171 |
| 33 | $CH_3$ | $CH_3$ | n-$C_3H_7$ | 168-170 |
| 34 | $CH_3$ | $CH_3$ | cyclohexyl | 176-178 |
| 35 | $CH_3$ | n-$C_4H_9$ | $C_2H_5$ | 87-89 |
| 36 | $C_2H_5$ | $CH_3$ | $CH_3$ | 143-146 |
| 37 | $C_2H_5$ | $CH_3$ | n-$C_4H_9$ | 121-123 |
| 38 | $C_2H_5$ | $C_2H_5$ | t-$C_4H_9$ | 116-118 |
| 39 | $C_2H_5$ | n-$C_3H_7$ | $CH_3$ | 155-157 |
| 40 | $C_2H_5$ | n-$C_3H_7$ | n-$C_3H_7$ | 65-67 |
| 41 | $C_2H_5$ | n-$C_3H_7$ | iso-$C_3H_7$ | 84-86 |
| 42 | $C_2H_5$ | n-$C_3H_7$ | n-$C_4H_9$ | 75-77 |
| 43 | $C_2H_5$ | iso-$C_3H_7$ | $C_2H_5$ | 75-77 |
| 44 | n-$C_3H_7$ | $C_2H_5$ | n-$C_3H_7$ | 62-64 |
| 45 | t-$C_4H_9$ | $CH_3$ | $C_2H_5$ | 114° |
| 46 | $CH_3$ | $CH_3$ | $CH_3$ | 156-159 |
| 47 | $CH_3$ | $CH_3$ | iso-$C_3H_7$ | 162-164 |

| Example No | $R^1$ | $R^{14}$ | $R^5$ | m.p. (°C) |
|---|---|---|---|---|
| 48 | $CH_3$ | n-$C_3H_7$ | n-$C_3H_7$ | 92-94 |
| 49 | $CH_3$ | n-$C_3H_7$ | iso-$C_3H_7$ | 104-106 |
| 50 | $C_2H_5$ | iso-$C_3H_7$ | n-$C_3H_7$ | 103-104 |
| 51 | $C_2H_5$ | iso-$C_3H_7$ | iso-$C_3H_7$ | 134-136 |
| 52 | n-$C_3H_7$ | $C_2H_5$ | iso-$C_3H_7$ | 74-76 |
| 53 | t-$C_4H_9$ | $CH_3$ | n-$C_3H_7$ | 174-175 |

Example 54

(1-ethoxypropylidene)malononitrile (70 parts) was added slowly to a stirred solution of hydroxylamine hydrochloride (40 parts) and sodium hydroxide (30 parts) in water (200 parts). The mixture was stirred for four hours, keeping the temperature below 50°C. The crystalline solid was then filtered off and recrystallised from water to give 5-amino-4-cyano-3-ethylisoxazole (46.5 parts), melting point 139-141°C.

Analysis: Found: C, 52.49; H, 5.24; N, 30.41%

$C_6H_7N_3O$ requires: C, 52.54; H, 5.15; N,30.64%

A mixture of this compound (40 parts) and butyl isocyanate (45 parts) in a solution of triethylamine (4 parts) in toluene (350 parts) was heated under reflux for 18 hours. The mixture was evaporated to dryness and the residue recrystallised from ethanol to give 1-butyl-3-(4-cyano-3-ethyl-5-isoxazolyl)urea (10 parts), melting point 171-173°C.

Analysis: Found: C, 55.86; H, 7.07; N, 23.46%

$C_{11}H_{16}N_4O_2$ requires: C, 55.91; H, 6.83; N, 23.72%

Example 55

By a method analogous to that of Example 54 there was prepared 1-ethyl-3-(4-cyano-3-ethyl-5-isoxazolyl)urea, melting point 147-149°C.

Example 56

A mixture of 5-amino-3-methyl-4-nitroisoxazole (50 parts) and ethyl isocyanate (50 parts) in toluene (350 parts) was heated under reflux and triethylamine (5 parts) was added. The mixture was heated under reflux for 15 minutes and then cooled in ice. The solid was filtered and washed with light petroleum (b.p. 40-60°C) to give 5-(3-ethylureido)-3-methyl-4-nitroisoxazole (45 parts) m.p. 181-183°C.

| | | | | |
|---|---|---|---|---|
| Analysis: | Found: | C, 39.35; | H, 4.70; | N, 26.06 |
| $C_7H_{10}N_4O_4$ | requires: | C, 39.24; | H, 4.71; | N, 26.16 |

Examples 57-60

The following compounds of formula I in which $R^3$ and $R^4$ each represents a hydrogen atom were prepared by methods analogous to that of Example 56:

| $R^1$ | $R^2$ | $R^5$ | m.p. (°C) |
|---|---|---|---|
| $CH_3$ | $NO_2$ | 3-chlorophenyl | 205-207° |
| $CH_3$ | $NO_2$ | *n*-$C_3H_7$ | 176-178° |
| $CH_3$ | $NO_2$ | *iso*-$C_3H_7$ | 180-181° |
| $CH_3$ | $NO_2$ | *n*-$C_4H_9$ | 154-156° |

Examples 61-68

Seeds of peas (*Pisum sativum*), mustard (*Sinapis alba*), linseed

(*Linum usitatissimum*), ryegrass (*Lolium perenne*), sugar beet (*Beta vulgaris*), oat (*Avena sativa*) and french beans (*Phaseolus vulgaris*) were sown in aluminium pans 19cm long x 9.5cm wide x 5cm deep containing John Innes I potting compost. They were then watered and placed in a controlled environment room (temperature 22°C, relative humidity 65-85%, artificial illumination 14 hours per day at 13000 lux). Fourteen days after sowing, the seedlings were sprayed with aqueous suspensions of the compounds listed below together with 1000 parts per million of nonyl phenol/ethylene oxide condensation product as wetting agent at rates equivalent to 2.8 or 0.7 kg of compound in 450 litres per hectare. The treated plants were then returned to the controlled environment room for seven days and then visually assessed for any herbicidal or growth regulant response. All differences from untreated controls were scored on a scale from 0 to 100 in which 0 represents no effect and 100 represents complete kill. The results are summarised below:

| Compound (Product of Example No) | Rate (kg/ha) | Herbicidal Activity Peas | Mustard | Linseed | Ryegrass | Oats | Sugar -beet | French Beans |
|---|---|---|---|---|---|---|---|---|
| 1 | 2.8 | 80 | 100 | 90 | 90 | 90 | 100 | 90 |
| | 0.7 | 30 | 100 | 60 | 60 | 40 | 100 | 30 |
| 10 | 2.8 | 5 | 5 | 70 | 5 | 5 | 70 | 0 |
| 11 | 2.8 | 50 | 100 | 90 | 60 | 50 | 100 | 30 |
| | 0.7 | 40 | 100 | 70 | 20 | 10 | 100 | 5 |
| 16 | 2.8 | 40 | 100 | 100 | 80 | 50 | 100 | 70 |
| | 0.7 | 15 | 40 | 70 | 5 | 5 | 30 | 5 |
| 17 | 2.8 | 50 | 90 | 100 | 40 | 30 | 100 | 100 |
| | 0.7 | 40 | 70 | 60 | 5 | 20 | 90 | 80 |
| 24 | 2.8 | 40 | 100 | 100 | 50 | 60 | 100 | 80 |
| | 0.7 | 30 | 90 | 90 | 40 | 5 | 100 | 10 |
| 25 | 2.8 | 80 | 100 | 100 | 70 | 70 | 100 | 100 |
| | 0.7 | 40 | 100 | 40 | 50 | 40 | 90 | 90 |
| 26 | 2.8 | 50 | 100 | 100 | 70 | 50 | 100 | 90 |
| | 0.7 | 40 | 100 | 80 | 30 | 40 | 100 | 30 |

Examples 69-74

In the same post-emergent test described in Examples 61-68, the following compounds gave the following results at 2.8 kg per ha on peas (P), mustard (M), linseed (L), ryegrass (R), oats (O), sugarbeet (S) and french beans (F):

| Compound | Herbicidal Activity P | M | L | R | S | O | F |
|---|---|---|---|---|---|---|---|
| Propyl 3-ethyl-5-(3-ethylureido)isoxazole-4-carboxylate | 70 | 100 | 100 | 100 | 100 | 90 | 100 |
| Propyl 3-ethyl-5-(3-butylureido)isoxazole-4-carboxylate | 40 | 100 | 100 | 80 | 100 | 30 | 90 |
| Butyl 3-methyl-5-(3-ethylureido)isoxazole-4-carboxylate | 50 | 100 | 100 | 90 | 100 | 50 | 100 |
| Ethyl 3-ethyl-5-(3-t-butylureido)isoxazole-4-carboxylate | 30 | 100 | 100 | 40 | 100 | 70 | 100 |
| Propyl 3-ethyl-5-(3-isopropylureido)isoxazole-4-carboxylate | 80 | 100 | 100 | 100 | 100 | 80 | 100 |
| Propyl 3-ethyl-5-(3-propylureido)isoxazole-4-carboxylate | 70 | 100 | 100 | 100 | 100 | 80 | 90 |

Examples 75-82

The compounds listed below, formulated as attaclay/sand dusts, were incorporated in John Innes I potting compost at a rate equivalent to 26 parts per million weight/volume of active ingredient to soil and placed in anodised aluminium pans, 19 cm long x 9.5 cm wide x 5.0 cm deep. This rate is approximately equivalent to a soil surface application of 11.2 kg of active ingredient per hectare cultivated to a depth of 5 cm. Seeds of peas (Pisum sativum), mustard (Sinapis alba), linseed (Linum usitatissimum), maize (Zea mays), oats (Avena sativa) and ryegrass (Lolium perenne) were then sown in the treated soil, watered and placed in a controlled environment

room (temperature 22°C, relative humidity 65-85°C, artificial illumination 14 hours per day at 13000 lux) for 21 days. The emergent plants were then visually assessed for any growth regulatory or herbicidal effects. All differences from untreated controls were scored on a scale from 0 to 100 in which 0 represents no effect and 100 represents complete kill. The results are summarised below:

| Compounds | Herbicidal effect | | | | | |
|---|---|---|---|---|---|---|
| (Product of Example No) | Peas | Mustard | Linseed | Ryegrass | Oats | Maize |
| 1 | 20 | 100 | 100 | 100 | 90 | 30 |
| 10 | 80 | 100 | 100 | 100 | 100 | 30 |
| 11 | 60 | 100 | 100 | 100 | 100 | 40 |
| 16 | 80 | 100 | 100 | 100 | 100 | 40 |
| 17 | 80 | 100 | 100 | 100 | 100 | 5 |
| 24 | 60 | 100 | 100 | 100 | 100 | 70 |
| 25 | 5 | 100 | 100 | 100 | 90 | 5 |
| 26 | 10 | 70 | 70 | 90 | 60 | 5 |

## Examples 83-87

In the same pre-emergent test described in Examples 75-82, the following compounds gave the following results on peas (P), mustard (M), linseed (L), maize (C), oats (O) and ryegrass (R):

| Compound | Herbicidal Effect P | M | L | C | O | R |
|---|---|---|---|---|---|---|
| Propyl 3-ethyl-5-(3-ethylureido) isoxazole-4-carboxylate | 40 | 100 | 100 | 30 | 100 | 100 |
| Butyl 3-methyl-5-(3-ethylureido) isoxazole-4-carboxylate | 40 | 100 | 100 | 20 | 100 | 100 |
| Ethyl 3-ethyl-5-(3-t-butyl-ureido) isoxazole-4-carboxylate | 30 | 100 | 100 | 70 | 100 | 100 |
| Propyl 3-ethyl-5-(3-isopropyl-ureido) isoxazole-4-carboxylate | 40 | 100 | 100 | 30 | 100 | 100 |
| Propyl 3-ethyl-5-(3-propyl-ureido) isoxazole-4-carboxylate | 40 | 100 | 100 | 10 | 100 | 100 |

Examples 88-94

The compounds:

(A) Methyl 3-ethyl-5-(3-isopropylureido) isoxazole-4-carboxylate;

(B) Ethyl 3-methyl-5-(3-methylureido) isoxazole-4-carboxylate;

(C) Ethyl 3-methyl-5-(3-ethylureido) isoxazole-4-carboxylate;

(D) Ethyl 3-ethyl-5-(3-ethylureido) isoxazole-4-carboxylate;

(E) Ethyl 3-ethyl-5-(3-isopropylureido) isoxazole-4-carboxylate;

(F) Propyl 3-methyl-5-(3-ethylureido) isoxazole-4-carboxylate; and

(G) Ethyl 3-methyl-5-(3-propylureido) isoxazole-4-carboxylate

were separately formulated as attaclay/sand dusts and incorporated in John Innes I potting compost at a rate equivalent to 6.5 parts per million weight/volume of active ingredient to soil and placed in anodised aluminium pans 19 cm long x 9.5 cm wide x 5.0 cm high. This is equivalent to a surface application of approximately 2.8 kg of active ingredient per hectare cultivated to a depth of 5 cm.

Seeds of the species listed below were sown in the treated soil, one species per pan, watered, and placed in a controlled environment room (22°C, relative humidity 65-85%, artificial illumination 14 hours per day at 17000 lux) for 21 days. The emergent plants were then assessed for any growth regulatory or herbicidal effects. All differences from an untreated control were scored on a scale from 0 to 100 in which 0 represents no effect and 100 represents complete kill. The results are summarised below:

| Species | Compound (A) | (B) | (C) | (D) | (E) | (F) | (G) |
|---|---|---|---|---|---|---|---|
| Chickweed (Stellaria media) | 100 | 100 | 100 | 100 | 100 | 100 | 90 |
| Mustard (Sinapis alba) | 100 | 100 | 100 | 100 | 100 | 100 | 90 |
| Cotton (Gossypium sp) | 20 | 5 | 0 | 0 | 15 | 0 | 0 |
| Tomato (Lycopersicon esculentum) | 100 | 100 | 100 | 90 | 100 | 100 | 90 |
| Fathen (Chenopodium album) | 100 | 100 | 90 | 100 | 100 | 100 | 90 |
| Carrot (Daucus carota) | 90 | 100 | 100 | 100 | 90 | 100 | 80 |
| Wheat (Triticum aestivum) | 20 | 90 | 70 | 40 | 70 | 50 | 0 |
| Barley (Hordeum vulgare) | 30 | 90 | 90 | 90 | 90 | 90 | 0 |
| Wild Oat (Avena fatua) | 70 | 90 | 100 | 90 | 100 | 100 | 30 |
| Blackgrass (Alopecurus myosuroides) | 70 | 100 | 90 | 70 | 100 | 100 | 40 |

Examples 95-98

Seeds of the various monocotyledon species listed below were sown in anodised aluminium pans 19 cm long x 9.5 cm wide x 5.0 cm

deep containing John Innes I potting compost. They were then watered and placed in a controlled environment room (22°C, relative humidity 65-85%, artificial illumination 14 hours per day at 17000 lux). Fourteen days after sowing, the seedlings were given a foliar spray of

(A) Ethyl 3-ethyl-5-(3-propylureido)isoxazole-4-carboxylate;

(B) Ethyl 3-ethyl-5-(3-butylureido)isoxazole-4-carboxylate;

(C) Propyl 3-ethyl-5-(3-ethylureido)isoxazole-4-carboxylate; or

(D) Ethyl 3-ethyl-5-(3-ethylureido)isoxazole-4-carboxylate;

formulated as aqueous suspensions together with 2000 parts per million of nonyl phenol/ethylene oxide condensation product as wetting agent at rates equivalent to 1.4 or 0.7 kg of compound in 450 litres per hectare. The treated plants were returned to the controlled environment room for 14 days and then visually assessed for any growth regulatory or herbicidal effect. All differences from an untreated control were scored on a scale from 0 to 100 in which 0 signifies no effect and 100 signifies complete kill. The results are summarised below:

| | | Compound | | | | | | | |
|---|---|---|---|---|---|---|---|---|---|
| | | (A) | | (B) | | (C) | | (D) | |
| Species | Dosage Rate kg/ha | 1.4 | 0.7 | 1.4 | 0.7 | 1.4 | 0.7 | 1.4 | 0.7 |
| Wheat (Triticum aestivum) | | 30 | - | 20 | - | 30 | - | 30 | - |
| Barley (Hordeum vulgare) | | 20 | - | 20 | - | 30 | - | 30 | - |
| Wild oat (Avena fatua) | | 40 | - | 30 | - | 70 | - | 50 | - |
| Blackgrass (Alopecurus myosuroides) | | 50 | - | 70 | - | 80 | - | 70 | - |
| Barnyardgrass (Echinochloa crus-galli) | | 30 | - | 30 | - | 70 | - | 30 | - |
| Crabgrass (Digitaria sanguinalis) | | 70 | - | 70 | - | 100 | - | 70 | - |
| Chickweed (Stellaria media) | | - | 90 | - | 100 | - | 100 | - | 100 |
| Mayweed (Matricaria sp) | | - | 100 | - | 100 | - | 100 | - | 100 |
| Cleavers (Galium aporine) | | - | 100 | - | 100 | - | 100 | - | 100 |
| Pale persicaria (Polygonum lopathifolium) | | - | 100 | - | 100 | - | 90 | - | 100 |
| Fathen (Chenopodium album) | | - | 100 | - | 100 | - | 100 | - | 100 |
| Corn marigold (Chrysanthemum regetum) | | - | 100 | - | 100 | - | 100 | - | 100 |
| Pigweed (Amaranthus retroflexus) | | - | 100 | - | 100 | - | 100 | - | 100 |

Examples 99-102

Each of the compounds listed in the Table below was admixed as a 50% wettable powder into water, together with 0.1% of nonyl phenolethylene oxide condensate as wetting agent based on the spray volume, and was sprayed at 0.5 or 1.0 kg of the compound per hectare onto the following crop and weed species at the growth stages specified:

Winter Wheat - variety Hobbit - *Triticum aestivium* - $2\frac{1}{2}$ leaf

Mayweed - *Tripleurospermum maritimum* - $4\frac{1}{2}$ leaf

Chickweed - *Stellaria media* - $3\frac{1}{2}$ pairs of leaves and 2 shoots

Speedwell - *Veronica persica* - $2-2\frac{1}{2}$ pairs of leaves

Pale persicaria - *Polygonum lapathifolium* - $1\frac{1}{4}-1\frac{1}{2}$ leaf

The plant species were growing in a greenhouse in 11cm round pots in sterilized loam. Three replicates were carried out per treatment. Three weeks after treatment, the herbicidal effect was assessed on a scale on which 0 represents no effect and 100 represents total kill. The results were:

| Compound | Rate, kg/ha | Wheat | Mayweed | Chickweed | Speedwell | Cleavers | Pale persicaria |
|---|---|---|---|---|---|---|---|
| Propyl 3-methyl-5-(3-butylureido) isoxazole-4-carboxylate | 0.5 | 0 | 65 | 65 | 65 | 65 | 80 |
| | 1.0 | 0 | 75 | 94 | 92 | 75 | 98 |
| Ethyl 3-ethyl-5-(3-butylureido) isoxazole-4-carboxylate | 0.5 | 0 | 65 | 80 | 92 | 50 | 80 |
| | 1.0 | 10 | 75 | 93 | 93 | 80 | 95 |
| Ethyl 3-ethyl-5-(3-propylureido) isoxazole-4-carboxylate | 0.5 | 2 | 80 | 95 | 95 | 35 | 100 |
| | 1.0 | 8 | 94 | 100 | 100 | 60 | 100 |
| Ethyl 3-ethyl-5-(3-isopropylureido) isoxazole-4-carboxylate | 0.5 | 0 | 55 | 75 | 92 | 10 | 92 |
| | 1.0 | 5 | 90 | 95 | 95 | 10 | 95 |

Example 103

A 50% wettable powder was prepared by mixing and micronising the following:

| | |
|---|---|
| Ethyl 3-ethyl-5-(3-butylureido)isoxazole-4-carboxylate | 50% |
| Reax 45L (combined wetting and dispersing agent based on lignin sulphonate *ex* Westvaco Corp) | 5% |
| China clay | 45% |

Example 104

A 50% wettable powder was prepared by mixing and micronising the following:

| | |
|---|---|
| Ethyl 3-ethyl-5-(3-propylureido)isoxazole-4-carboxylate | 50% |
| Reax 45L | 5% |
| China clay | 45% |

Example 105

A 25% wettable powder was prepared by mixing and micronising the following:

| | |
|---|---|
| Ethyl 3-ethyl-5-(3-isopropylureido)isoxazole-4-carboxylate | 25% |
| Arkopon T highly conc (sodium oleyl-N-methyltauride *ex* Hoechst) | 5% |
| Sopropon T36 (polycarboxylate dispersing agent *ex* Rhone-Poulenc) | 2% |
| China clay | 68% |

0002881

Examples 106 and 107

(A) Ethyl 3-ethyl-5-(3-butylureido)isoxazole-4-carboxylate; and

(B) Ethyl 3-ethyl-5-(3-propylureido)isoxazole-4-carboxylate,

formulated as in Examples 103 and 104 respectively were sprayed late post-emergence in the field onto the crop and weed species listed in the table below at the rate of active ingredient which is also shown in the table below. After 3 weeks, the herbicidal effect was assessed on each species on a scale from 0 to 10 on which 0 represents no effect and 10 represents complete kill.

The results are as follows:

| Chemical | A | | | B | | |
|---|---|---|---|---|---|---|
| Rate, kg/ha | 0.25 | 0.75 | 2.25 | 0.25 | 0.75 | 2.25 |
| Species Sown | | | | | | |
| Spring Wheat - M Dove | 2 | 2 | 2.5 | 1 | 1 | 2 |
| Spring Barley - | | | | | | |
| M Mink | 1 | 1.5 | 2 | 0.5 | 1.5 | 2 |
| G Promise | 1 | 1.5 | 2.5 | 1 | 2 | 2.5 |
| Spring Oat | 0 | 0 | 2 | 0 | 0 | 0 |
| Winter Rye | 0.5 | 0.5 | 1.5 | 0.5 | 1 | 1.5 |
| Wild Oat | 1.5 | 1.5 | 8.5 | 3 | 5 | 5.5 |
| Pale Persicaria | 7 | 8.5 | 10 | 5.5 | 9 | 9.5 |
| B Speedwell | 6.5 | 8 | 8.5 | 6.5 | 8 | 7.5 |
| S Mayweed | 7.5 | 7.5 | 8 | 5.5 | 7.5 | 8 |
| Chickweed | 2 | 6 | 7.5 | 3 | 5.5 | 7 |
| Cleavers | 1.5 | 1.5 | 2 | 1.5 | 2.5 | 2 |
| Blackgrass | 2 | 3 | 3 | 2.5 | 4 | 3 |

| Chemical | A | | | B | | |
|---|---|---|---|---|---|---|
| Rate, kg/ha | 0.25 | 0.75 | 2.25 | 0.25 | 0.75 | 2.25 |
| Indigenous | | | | | | |
| Black Bindweed | 8.5 | 9 | 9.5 | 5.5 | 8.5 | 9 |
| Fat Hen | 9.5 | 10 | 10 | 10 | 10 | 10 |
| Knotgrass | 6 | 5.5 | 7.5 | 6 | 6.5 | 6.5 |
| Scarlet Pimpernel | 7 | 9 | 8.5 | 6 | 10 | 10 |
| Charlock | 8.5 | 9.5 | 9.5 | 6.5 | 9.5 | 10 |
| Groundsel | 6.5 | 9.5 | 10 | 3.5 | 7.5 | 9.5 |
| White Campion | 3 | 5 | 9.5 | 2.5 | 8.5 | 6.5 |

Example 108

The compound ethyl 3-ethyl-5-(3-isopropylureido)isoxazole-4-carboxylate, formulated as in Example 105, was sprayed per-emergence in the field where the crop and weed species listed in the table below were growing. The rate of application was 1.11 kg of the chemical per hectare. After 7 and 13 weeks, the herbicidal effect was assessed on each species on a scale from 0 to 10 on which 0 represents no effect and 10 represents complete kill.

The results are as follows:

| | 7 Weeks | 13 Weeks |
|---|---|---|
| Species Sown | | |
| Spring Barley - | | |
| M Mink | 1 | 2 |
| G Promise | 2 | 2 |
| Spring Oat | 2 | 1.5 |
| Winter Rye | 2 | 2 |
| Wild Oat | 5 | 9.5 |
| Mustard | 7 | 2 |
| Pale Persicaria | 8 | 8.5 |
| B Speedwell | 9.5 | 9.5 |
| S Mayweed | 9.5 | 9 |
| Chickweed | 9.5 | 9.5 |
| Cleavers | 7 | 2 |
| Blackgrass | 5 | 4 |
| Indigenous | | |
| Charlock | - | 9 |
| Fat Hen | - | 10 |
| Black Bindweed | 7.5 | 8.5 |
| Couch | 2 | 3 |
| Knotgrass | - | 8 |
| Creeping Thistle | 1.5 | 3 |

Examples 109 and 110

The compounds

(a) Ethyl 3-ethyl-5-(3-butylureido)isoxazole-4-carboxylate; and

(B) Ethyl 3-ethyl-5-(3-propylureido)isoxazole-4-carboxylate,

formulated as in Examples 103 and 104 respectively, were sprayed post-emergence onto the crop and weed species listed in the table below at the rate of active ingredient which is also shown in the table below. After 6 weeks, the herbicidal effect was assessed on a scale from 0 to 10 on which 0 represents no effect and 10 represents complete kill.

The results are as follows:

| Species \ Chemical Rate per ha | A 0.3 kg | B 0.3 kg | A 1 kg | B 1 kg |
|---|---|---|---|---|
| Maize | 2 | 1.5 | 3 | 4 |
| Pearl Millet | 8 | 8.5 | 9.5 | 9 |
| Barnyardgrass | 2 | 3.5 | 3 | 3.5 |
| Common Millet | 5.5 | 9 | 9 | 9.5 |
| Foxtail Millet | 5 | 6.5 | 7 | 8 |
| Sorghum | 3.5 | 2.5 | 4 | 2.5 |
| Rice | 1 | 1 | 1.5 | 1 |
| Wheat - | | | | |
| M Dove | 2 | 6.5 | 6 | 7.5 |
| Sappo | 2 | 5 | 5.5 | 5 |
| Barley - | | | | |
| Golden Promise | 0 | 2.5 | 2 | 3 |
| M Mink | 1.5 | 2 | 2 | 2.5 |
| Oats | 2.5 | 1.5 | 3 | 1.5 |
| Rye | 6 | 4 | 4 | 5 |
| Wild Oats | 5 | 6 | 3.5 | 5.5 |
| Perenial Ryegrass | 4.5 | 5.5 | 5 | 5.5 |
| Italian Ryegrass | 2 | 4.5 | 4 | 5 |

| Species \ Chemical Rate per ha | A 0.3 kg | B 0.3 kg | A 1 kg | B 1 kg |
|---|---|---|---|---|
| Oil Seed Rape - | | | | |
| Erglu | 5.5 | 9 | - | - |
| M Haplona | 8.5 | 8.5 | 9.5 | 9.5 |
| Mustard | 5 | 5.5 | 8 | 7 |
| Charlock | 9 | 9 | 9.5 | 9.5 |
| Velvetleaf | 7.5 | 8 | 9.5 | 9 |
| Buckwheat | 9.5 | 9.5 | 9.5 | 10 |
| Sugarbeet | 8.5 | 9.5 | 9.5 | 10 |
| Soyabeans | 4 | 2.5 | 5.5 | 4.5 |
| Peanuts | 5.5 | 5.5 | 4.5 | 4 |
| Peas | 2 | 2 | 3 | 3 |
| Navy Beans | 1.5 | 5.5 | 3 | 7 |
| Sunflowers | 2 | 5 | 7.5 | 7 |
| Potatoes | 0.5 | 1.5 | 1 | 1.5 |
| Timothy | 2 | 2.5 | 4 | 7.5 |
| Cotton | 2.5 | 2.5 | 3 | 3 |
| Pigweed | 7.5 | 7 | 9 | 9.5 |
| Koelia | 6.5 | 8.5 | 9.5 | 9.5 |
| Fathen | 8.5 | 9.5 | 9.5 | 9.5 |
| Purslane | 8.5 | 9 | 9.5 | 9 |
| Chickweed | 9 | 9 | 9.5 | 9.5 |
| S Mayweed | 9 | 9.5 | 10 | 9.5 |
| Rayless Mayweed | 5.5 | 8 | 9.5 | 9.5 |
| B Speedwell | 8.5 | 8.5 | 9 | 10 |

| Species \ Chemical Rate per ha | A 0.3 kg | B 0.3 kg | A 1 kg | B 1 kg |
|---|---|---|---|---|
| Field Pansy | 7.5 | 7.5 | 9.5 | 9 |
| Corn Marigold | 5 | 9.5 | 9.5 | 9.5 |
| Pale Persicaria | 9 | 9.5 | 10 | 10 |
| Penn Smartweed | 5.5 | 5 | 8.5 | 7.5 |
| Sicklepod | 4 | 7 | 3.5 | 4.5 |
| Annual Morning Glory | 2 | 3 | 2.5 | 3 |
| Jimsonweed | 9.5 | 9 | 9 | 9 |
| Yorkshire Fog | 1 | 1.5 | 1.5 | 1.5 |
| Couch (seed) | 3 | 2 | 2.5 | 2 |
| Annual Meadow Grass | 2.5 | 2.5 | 2 | 3.5 |
| Sprangletop | 0.5 | 2 | 2.5 | 3 |
| G Foxtail | 1 | 2 | 2.5 | 4 |
| Johnsongrass | 2.5 | 2.5 | 3.5 | 5.5 |
| Crabgrass | 1.5 | 3 | 2.5 | 5 |
| Onions | 2 | 2 | 2.5 | 3 |
| Downy Brome | 4 | 4.5 | 3.5 | 4 |
| Yellow Foxtail | 3.5 | 3 | 2.5 | 3.5 |
| Canary grass | 3.5 | 3.5 | 4 | 5.5 |
| Blackgrass | 7 | 8 | 6 | 7 |

Example 111

A mixture of 5-[3-isopropylureido]-3-methyl-4-nitro-isoxazole (52 parts) and oxalyl chloride (70 parts) in toluene (500 parts)

was heated under reflux for 5½ hours. The solution was evaporated and the residue was recrystallised from ethanol to give 5-[3-isopropyl-2,4,5-trioxoimidazolidin-1-yl]-3-methyl-4-nitroisoxazole (34 parts), melting point 124-126°C.

| | | | | |
|---|---|---|---|---|
| Analysis | Found: | C, 42.30; | H, 4.00; | N, 20.09% |
| $C_{10}H_{10}N_4O_6$ | requires: | C, 42.56; | H, 3.57; | N, 19.85% |

Example 112

A mixture of methyl 3-ethyl-5-[3-cyclohexylureido]-isoxazole-4-carboxylate (40 parts) and oxalyl chloride (40 parts) in toluene (400 parts) was heated under reflux for 2½ hours. The solution was evaporated and the residue was recrystallised from ethanol to give methyl 5-[3-cyclohexyl-2,4,5-trioxoimidazolidin-1-yl]-3-ethylisoxazole-4-carboxylate (28 parts), melting point 120-122°C.

| | | | | |
|---|---|---|---|---|
| Analysis | Found: | C, 55.29; | H, 5.75; | N, 11.94% |
| $C_{16}H_{19}N_3O_6$ | requires: | C, 55.01; | H, 5.48; | N, 12.03% |

Examples 113-118

The following compounds were prepared by methods analogous to that of Example 112:

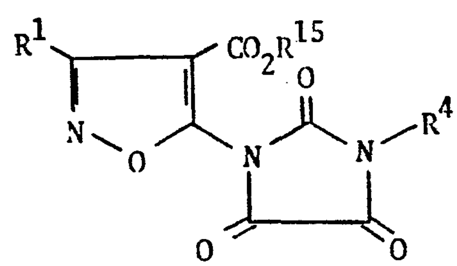

| $R^1$ | $R^{15}$ | $R^4$ | Melting Point ( $^oC$) |
|---|---|---|---|
| $CH_3$ | $CH_3$ | $C_2H_5$ | 112-114 |
| $CH_3$ | $CH_3$ | n-$C_3H_7$ | 85-86 |
| $CH_3$ | $CH_3$ | iso-$C_3H_7$ | 104-106 |
| $CH_3$ | $C_2H_5$ | $C_2H_5$ | 124-125 |
| $C_2H_5$ | $CH_3$ | iso-$C_3H_7$ | 67-69 |
| $C_2H_5$ | $C_2H_5$ | n-$C_3H_7$ | 45-47 |

Example 119

A solution of pivalaldehyde (200 parts) in ethanol (400 parts) was added to a solution of hydroxylamine hydrochloride (320 parts) and sodium hydroxide (185 parts) in water (650 parts) and the solution was heated under reflux for ½ hour. The mixture was cooled and extracted with ether (3 x 400 mls). The ether extracts were dried, evaporated and distilled under vacuum to give pivalaldehyde oxime (130 parts), boiling point 68-74$^oC$/30 mm Hg.

Example 120

A solution of chlorine (41 parts) in carbon tetrachloride (300 mls) was added slowly with stirring to a solution of pivaldehyde oxime (58 parts) in carbon tetrachloride (300 ml). The temperature was maintained at 10$^oC$ throughout the addition and the stirring was then maintained for a further 22 hours. Evaporation of the mixture gave pivalohydroximoyl chloride (92.7 parts).

Example 121

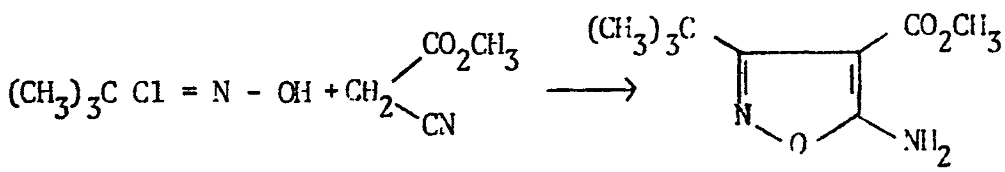

A solution of sodium methoxide (44.5 parts) in methanol (300 mls) was treated with methyl cyanoacetate (67.7 parts), and pivalohydroximoyl chloride (92.7 parts) was added slowly with stirring. The temperature was maintained in the range 10-15°C during the addition. The mixture was stirred for a further 3 hours at 25°C and then evaporated to dryness. The residue was recrystallised from cyclohexane to give methyl 5-amino-3-tert-butyl-isoxazole-4-carboxylate (46.8 parts), melting point 103-106°C.

Example 122

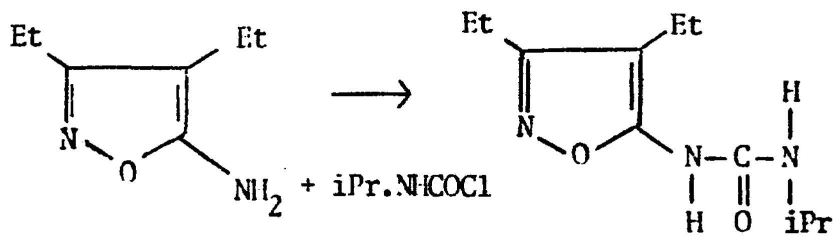

The isoxazole amine in pyridine and triethylamine at 0°C is treated with the carbamoyl chloride, and the resulting mixture is stirred at room temperature for 10 days. Evaporation of the solvents gives a residue which is treated with water giving ethyl 3-ethyl-5-(3-isopropylureido)isoxazole-4-carboxylate.

Example 123

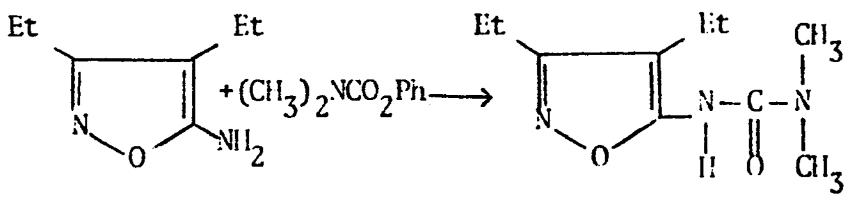

The isoxazoleamine and phenyl N,N-dimethylcarbamate in toluene are heated under reflux for 24 hours. The toluene is evaporated and the residue is recrystallised giving the required isoxazole urea.

Example 124

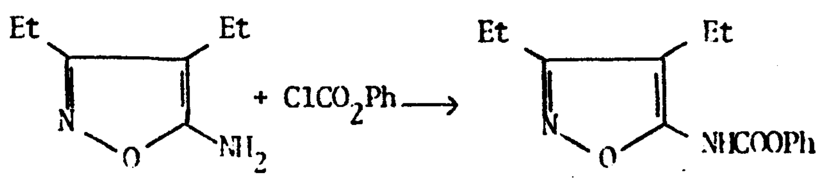

The isoxazoleamine in triethylamine and pyridine at 0°C is treated with phenyl chloroformate and the mixture is stirred at room temperature for seven days. The excess reagents are removed giving the phenyl carbamate.

Example 125

The phenyl carbamate prepared in Example 124 and butylamine in toluene are heated under reflux for 24 hours. The solvents are evaporated and the residue is recrystallised giving ethyl 3-ethyl-5-(3-n-butylureido)isoxazole-4-carboxylate.

Example 126

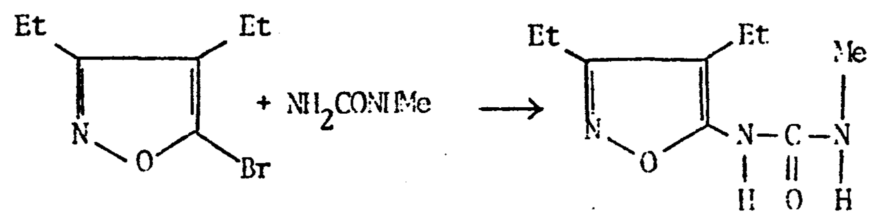

The 5-bromoisoxazole and methylurea are heated together in a nitrogen atmosphere at 150°C for six hours. The residue is triturated with water giving the required isoxazolyl urea.

Example 127

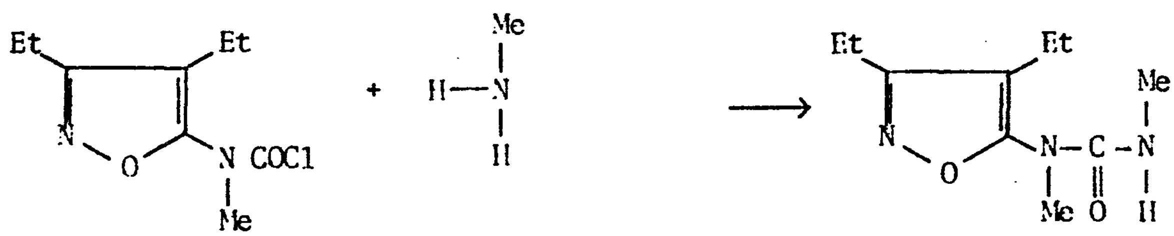

The isoxazolyl-methyl-carbamoyl chloride is prepared by the reaction of isoxazolyl-methyl-amine in chloroform with phosgene gas at a temperature of approximately 50°. The carbamoyl chloride is added at room temperature to a solution of methylamine in toluene. The mixture is stirred at room temperature for 2 hours and then at 50°C for 4 hours. Evaporation of the excess reagents and treatment of the residue with water gives the required urea.

Example 128

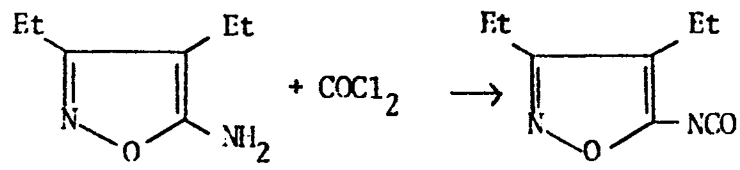

The 5-aminoisoxazole is dissolved in toluene, and hydrogen chloride is introduced at room temperature for 30 minutes. The mixture is agitated for 1½ hours and then the excess hydrogen chloride gas is removed under vacuum. Toluene is added and phosgene gas is blown into the reaction mixture at 50-70°C for 3-5 hours. The mixture is allowed to stand overnight at room temperature, and the required product is isolated by removing excess reagents under vacuum.

Example 129

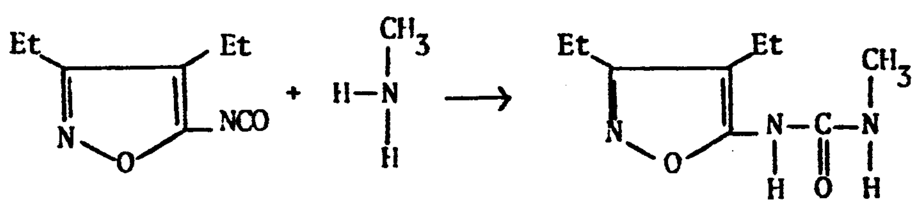

The 5-isocyanatoisoxazole prepared in Example 128 is added at room temperature to a solution of methylamine in toluene. The mixture is stirred at room temperature for 2 hours and then 4 hours at 50°C. Working up gives the required 5 isoxazolyl methyl urea.

Example 130

A solution of ethyl 3-ethyl-5-(3-butylureido)isoxazole-4-carboxylate (5g) in tetrahydroguran (50 mls) is added slowly to a stirred suspension of sodium hydride (0.56 g) (80% dispersion in oil) in tetrahydrofuran (100 mls). The resulting suspension of sodium salt is stirred for 30 minutes at room temperature. Methyl iodide (1.2 mls) is added cautiously and the mixture is then heated under reflux for 2 hours. The solvent is evaporated and the residue is treated with water giving ethyl 3-ethyl-5-(3-butyl-1-methylureido)isoxazole-4-carboxylate.

Example 131

A solution of ethyl 3-ethyl-5-(3-butylureido)isoxazole-4-carboxylate (5g) in tetrahydrofuran (50 mls) is added slowly to a stirred suspension of sodium hydride (0.56 g) (80% dispersion in oil) in tetrahydrofuran (100 mls). The resulting suspension of sodium salt is stirred for 30 minutes at room temperature. Acetic anhydride (1.7 mls) is added cautiously and the mixture is then heated under reflux for 2 hours. The solvent is evaporated and the residue is treated with water giving ethyl 3-ethyl-5-(1-acetyl-3-butylureido)isoxazole-4-carboxylate.

Example 132

A solution of ethyl 3-ethyl-5-(3-isopropylureido)isoxazole-4-carboxylate (5 g) in methanol (20 mls) is added to a stirred solution of sodium methoxide (1.2 g) in methanol (20 mls) and the mixture is stirred at room temperature for 1 hour. The solid

is filtered and washed with a little cold methanol giving the sodium salt of ethyl 3-ethyl-5-(3-isopropylureido)isoxazole-4-carboxylate.

Example 133

A solution of the sodium salt of ethyl 3-ethyl-5-(3-isopropylureido)isoxazole-4-carboxylate (5g) in water (50 mls) is acidified to pH1 with concentrated hydrochloric acid. The solid is filtered and washed well with water giving ethyl 3-ethyl-5-(3-isopropylureido)isoxazole-4-carboxylate.

Examples 134-139

Aqueous suspensions containing 500, 125 or 31 ppm (parts per million) of the compounds listed below together with 125 ppm of nonyl phenol/ethylene oxide condensate as wetting agent were applied to the soil surrounding the roots (SS in the table below) or to the leaves (P in the table below) of wheat plants having one fully expanded leaf. After 24 hours, the treated plants were innoculated with an aqueous suspension of spores of the disease organism wheat brown leaf rust (Puccinia recondita). The plants were then placed in an atmosphere of 100% relative humidity at 18°C for 24 hours, and then transferred to a controlled environment room (temperature 18°C, relative humidity 80-90%) for 12 days. Then the incidence of fungal disease was assessed by comparison with controls sprayed with a solution of the wetting agent alone. Assessment was on a 0-8 scale where

| | | | | | | |
|---|---|---|---|---|---|---|
| 8 represents | >94% | control | of | the | disease |
| 7 " | 85-94% | " | " | " | " |
| 6 " | 75-84% | " | " | " | " |
| 5 " | 65-74% | " | " | " | " |
| 4 " | 55-64% | " | " | " | " |
| 3 " | 45-54% | " | " | " | " |
| 2 " | 35-44% | " | " | " | " |
| 1 " | < 20% | " | " | " | " |

The results are as follows:

| Compound of Example | | Rust Control Score | | |
|---|---|---|---|---|
| | | 500 ppm | 125 ppm | 31 ppm |
| 13 | SS | 7 | 3 | - |
| 36 | SS | 8 | - | - |
| 29 | SS | 6 | 1 | - |
| 24 | P | - | 3 | - |
| | SS | 6 | 5 | - |
| 41 | P | - | 3 | - |
| | SS | 8 | 8 | - |
| 42 | P | - | - | 8 |

Example 140

Aqueous suspensions containing 125 ppm of the compound of Example 41 together with 125 ppm of nonylphenol/ethylene oxide condensate as wetting agent were sprayed until the foliage was completely wetted on to young French bean plants (*Phaseolus vulgaris*)

having two fully expanded leaves. After 24 hours, the treated plants were inoculated with an aqueous suspension of spore of the disease organism bean rust (Uromyces phaseoli). The plants were then placed in a water saturated atmosphere for 24 hours and then kept in a controlled environment room (temperature $18^o$, relative humidity 80-90%) for 14 days, after which the incidence of fungal disease was measured. The percentage disease control in comparison with plants sprayed with a solution of the wetting agent alone was found to be 3 on the scale set out in Examples 134-139.

Examples 141-143

Aqueous suspensions containing 500 parts per million (ppm) of the compounds listed below and 125 ppm of nonylphenol/ethylene oxide condensate as wetting agent were applied as combined foliar sprays and soil drenches to young potato plants having seven fully expanded leaves. After 24 hours, the treated plants were inoculated with an aqueous suspension of sporangia of the disease organism potato blight (Phytophthora infestans). The plants were then placed in a water saturated atmosphere for 24 hours and then kept in a controlled environment room (temperature $18^o$, relative humidity 80-90%) for five days, after which the incidence of fungal disease was measured. The percentage disease control in comparison with plants sprayed with a solution of the wetting agent alone is tabulated below. Assessment was on the scale set out in Examples 134-139.

| Compound of Example | Potato Blight Control Score |
|---|---|
| 17 | 6 |
| 44 | 6 |
| 14 | 4 |

Examples 144-150

Aqueous acetone solutions or suspensions containing 500, 125 or 31 ppm of the compounds listed below together with 125 ppm of a wetting agent were applied to the leaves of young vine plants having five fully expanded leaves. The treated plants, together with controls treated with wetting agent alone, were inoculated 24 hours after the chemical application with an aqueous suspension of sporangia of the disease organism vine downy mildew, Plasmopara viticola. The plants were then placed in a water saturated atmosphere until the disease incidence was measured twelve days later in comparison with the controls treated with the solution of wetting agent alone. Assessment was on the scale set out in Examples 134-139. The results are as follows:

| Compound of Example | Downy Mildew Control Score | | |
|---|---|---|---|
| | 500 ppm | 125 ppm | 31 ppm |
| 48 | 7 | - | - |
| 17 | - | 6 | - |
| 25 | 8 | 5 | - |
| 26 | 8 | 5 | - |
| 41 | 8 | 7 | 4 |
| 42 | 7 | 6 | - |
| 24 | 4 | - | - |

Examples 151-153

Aqueous suspensions containing 500 ppm of the compounds listed below together with 125 ppm of nonyl phenol/ethylene oxide condensate as wetting agent were sprayed until the foliage was completely wetted on to rice plants having 2 fully expanded leaves. After 24 hours, the treated plants were inoculated with an aqueous suspension of spores of the disease organism rice blast (*Pyricularia oryzae*). The inoculated plants were placed in an atmosphere of 80% relative humidity at 28°C for 7 days. The disease incidence was then measured on the scale set out in Examples 134-139 by comparison with controls sprayed with the wetting agent alone. The results are as follows:

| Compound of Example | Rice Blast Control |
|---|---|
| 41 | 7 |
| 52 | 7 |
| 59 | 7 |

Examples 154-156

Aqueous acetone solutions or suspensions containing 125 or 31 mg per litre of the compounds listed below together with 125 mg per litre of a non-ionic wetting agent were sprayed on the foliage of young barley plants (3 leaf stage). The treated plants, together with controls treated with wetting agent alone, were inoculated 24 hours after the chemical application with spores of cereal powdery mildew, Erysiphe graminis. The plants were then placed in a controlled environment room held at 16°C and 60% relative humidity for 10 days when the percentage of disease control was assessed. Assessment was on the scale set out in Examples 134-139 in comparison with the controls treated with the wetting agent alone. The results are as follows:

| Compound of Example | Powdery Mildew Control 125 ppm | 31 ppm |
|---|---|---|
| 17 | 7 | - |
| 26 | 6 | - |
| 42 | - | 8 |

CLAIMS:

1. A compound which is a 5-isoxazolyl urea of formula

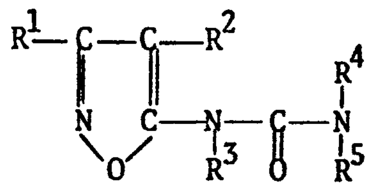

I

or a salt thereof,

wherein $R^1$ represents a hydrogen or halogen atom or a nitro, amino, substituted amino, alkyl, substituted alkyl, cycloalkyl, aryl, substituted aryl, aralkyl, or substituted aralkyl, group, or a group of formula $-XR^6$ or $-COOR^{15}$ in which X represents oxygen, sulphur, sulphinyl or sulphonyl, $R^6$ represents alkyl or aryl, and $R^{15}$ represents a hydrogen atom or an alkyl, substituted alkyl, aryl, substituted aryl, or cycloalkyl, group;

$R^2$ represents cyano, nitro, carboxy, carboxy ester, carboxyamide, carboxyamide whose amide group is substituted, or a group of formula $-\overset{O}{\overset{\|}{C}}-N\bigcirc$ in which $N\bigcirc$ represents a 5-7 ring membered heterocyclic radical which optionally may be substituted;

$R^3$ represents hydrogen, alkyl or a group of formula $-\overset{O}{\overset{\|}{C}}-R^7$ in which $R^7$ represents alkyl, aryl, alkoxy or alkylthio;

$R^4$ represents hydrogen, alkyl or cycloalkyl;

$R^5$ represents hydrogen, alkyl, cycloalkyl, alkoxy, aryl or substituted aryl; or

$R^3$ and $R^5$ together represent alkylene, optionally unsaturated, optionally interrupted by one or more atoms selected from nitrogen,

oxygen and sulphur atoms, and optionally substituted.

2. A compound according to claim 1 wherein $R^1$ represents a hydrogen or halogen atom; nitro; amino substituted by one or two substituents selected from alkyl of 1-10 carbon atoms and alkylcarbonyl of 2-7 carbon atoms; alkyl of 1-10 carbon atoms; alkyl of 1-10 carbon atoms substituted by one or more substituents selected from halogen, alkoxy of 1-6 carbon atoms and nitro; cycloalkyl of 3-7 carbon atoms; phenyl; phenyl substituted by one or more substituents selected from halogen, alkoxy of 1-6 carbon atoms, nitro and alkyl of 1-10 carbon atoms; phenylalkyl of 7-10 carbon atoms; phenylalkyl of 7-10 carbon atoms whose phenyl group is substituted by one or more substituents selected from halogen, alkoxy of 1-6 carbon atoms, nitro and alkyl of 1-10 carbon atoms; or a group of formula $-XR^6$ or $-COOR^{15}$ in which X represents oxygen, sulphur, sulphinyl or sulphonyl, $R^6$ represents alkyl of 1-10 carbon atoms or phenyl, and $R^{15}$ represents a hydrogen atom; alkyl of 1-10 carbon atoms; alkyl of 1-10 carbon atoms substituted by one or more substituents selected from halogen, alkoxy of 1-6 carbon atoms and nitro; phenyl; phenyl substituted by one or more substituents selected from halogen, alkoxy of 1-6 carbon atoms, nitro and alkyl of 1-10 carbon atoms; or cycloalkyl of 3-7 carbon atoms;

$R^2$ represents cyano; nitro; carboxy; alkoxycarbonyl of 2-7 carbon atoms; phenoxycarbonyl whose phenyl group optionally may be substituted by one or more substituents selected from halogen, alkoxy of 1-6 carbon atoms, alkyl of 1-10 carbon atoms and nitro;

benzyloxycarbonyl; carboxyamide; carboxyamide whose amide group is substituted by one or two substituents selected from alkyl of 1-10 carbon atoms and alkylcarbonyl of 2-7 carbon atoms; or a group of formula $-\overset{O}{\overset{\|}{C}}-N\Big)$ in which $N\Big)$ represents a 5-7 ring membered heterocyclic group containing in the ring besides the nitrogen atom either none or one other hetero atom selected from nitrogen and oxygen, which group optionally may be substituted by alkyl of 1-10 carbon atoms;

$R^3$ represents hydrogen, alkyl of 1-10 carbon atoms or a group of formula $-\overset{O}{\overset{\|}{C}}-R^7$ in which $R^7$ represents alkyl of 1-10 carbon atoms, phenyl, alkoxy of 1-6 carbon atoms or alkylthio of 1-6 carbon atoms;

$R^4$ represents hydrogen, alkyl of 1-10 carbon atoms or cycloalkyl of 3-7 carbon atoms;

$R^5$ represents hydrogen, alkyl of 1-10 carbon atoms, cycloalkyl of 3-7 carbon atoms, alkoxy of 1-6 carbon atoms, phenyl, or phenyl substituted by one or more substituents selected from halogen, alkoxy of 1-6 carbon atoms, nitro and alkyl of 1-10 carbon atoms; or

$R^3$ and $R^5$ together represent an alkylene group of 1-5 carbon atoms, which group is optionally unsaturated, optionally interrupted by one or more atoms selected from nitrogen, oxygen and sulphur atoms, and optionally substituted by one or more groups selected from halogen, hydroxy, alkoxy of 1-6 carbon atoms, oxo and alkanoyloxy of 2-7 carbon atoms.

3. A compound according to claim 1 wherein $R^1$ represents alkyl of

1-4 carbon atoms, $R^2$ represents cyano, nitro or alkoxycarbonyl of 2-5 carbon atoms, $R^3$ represents hydrogen, $R^4$ represents hydrogen, alkyl of 1-4 carbon atoms or cyclohexyl, $R^5$ represents alkyl of 1-4 carbon atoms, cyclohexyl, 4-chlorophenyl or 3-chlorophenyl, or $R^3$ and $R^5$ together represent 2,4,5-trioxoimidazolidin-1-yl.

4. A compound according to claim 1 wherein the 5-isoxazolyl urea is specifically identified herein.

5. A compound according to any one of the preceding claims which is the 5-isoxazolyl urea itself.

6. Ethyl 3-ethyl-5-(3-isopropylureido)isoxazole-4-carboxylate; ethyl 3-ethyl-5-(3-n-butylureido)isoxazole-4-carboxylate; or ethyl 3-ethyl-5-(3-n-propylureido)isoxazole-4-carboxylate.

7. Ethyl 3-methyl-5-(3-ethylureido)isoxazole-4-carboxylate, n-propyl 3-methyl-5-(3-ethylureido)isoxazole-4-carboxylate, ethyl 3-methyl-5-(3-methylureido)isoxazole-4-carboxylate, n-propyl 3-ethyl-5-(3-ethylureido)isoxazole-4-carboxylate, n-propyl 3-ethyl-5-(3-n-butylureido)isoxozole-4-carboxylate, n-butyl 3-methyl-5-(3-ethylureido)isoxazole-4-carboxylate, ethyl 3-ethyl-5-(3-tert butylureido)isoxazole-4-carboxylate, n-propyl 3-ethyl-5-(3-isopropylureido)isoxazole-4-carboxylate, n-propyl 3-ethyl-5-(3-n-propylureido)isoxazole-4-carboxylate, n-propyl 3-methyl-5-(3-n-butylureido)isoxazole-4-carboxylate, or ethyl 3-ethyl-5-(3-ethylureido)isoxazole-4-carboxylate.

8. A process for preparing a compound which is a 5-isoxazolyl urea of formula

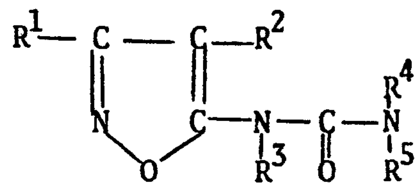

I

or a salt thereof,

wherein $R^1$ represents a hydrogen or halogen atom or a nitro, amino, substituted amino, alkyl, substituted alkyl, cycloalkyl, aryl, substituted aryl, aralkyl, or substituted aralkyl, group, or a group of formula $-XR^6$ or $-COOR^{15}$ in which X represents oxygen, sulphur, sulphinyl or sulphonyl, $R^6$ represents alkyl or aryl, and $R^{15}$ represents a hydrogen atom or an alkyl, substituted alkyl, aryl, substituted aryl, or cycloalkyl, group;

$R^2$ represents cyano, nitro, carboxy, carboxy ester, carboxyamide, carboxyamide whose amide group is substituted, or a group of formula $-C(=O)-N$ in which $N$ represents a 5-7 ring membered heterocyclic radical which optionally may be substituted;

$R^3$ represents hydrogen, alkyl or a group of formula $-C(=O)-R^7$ in which $R^7$ represents alkyl, aryl, alkoxy or alkylthio;

$R^4$ represents hydrogen, alkyl or cycloalkyl;

$R^5$ represents hydrogen, alkyl, cycloalkyl, alkoxy, aryl or substituted aryl; or

$R^3$ and $R^5$ together represent alkylene, optionally unsaturated, optionally interrupted by one or more atoms selected from nitrogen, oxygen and sulphur atoms, and optionally substituted, which process comprises:

(a) reacting a 5-aminoisoxazole of formula

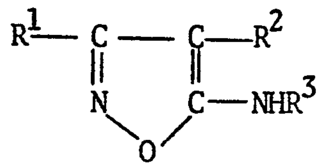

II

with a carbamoyl halide of formula $R^4R^5N\overset{O}{C}Z$, or reacting an isoxazolylcarbamyl halide of formula

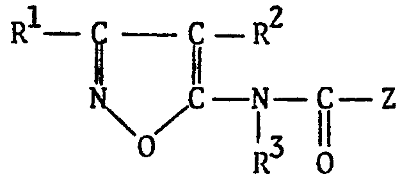

V

with an amine of formula $HNR^4R^5$, where Z represents a halogen atom;

(b) reacting a 5-aminocarboxyisoxazole of formula

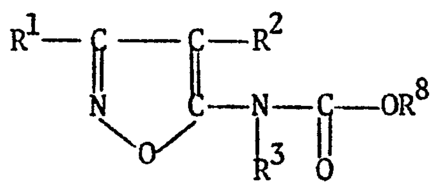

III

with the amine of formula $HNR^4R^5$, or reacting the 5-aminoisoxazole of formula II with a carbamate of formula $R^8O\text{-}\underset{O}{C}\text{-}NR^4R^5$, where $R^8$ represents phenyl or 2,4-dinitrophenyl;

(c) reacting the 5-aminoisoxazole of formula II with an isocyanate of formula $R^5NCO$ to product a compound in which $R^4$ represents hydrogen, or reacting a 5-isocyanatoisoxazole of formula

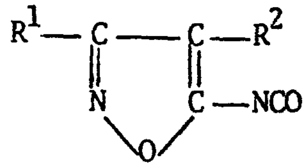

IV

with the amine of formula $HNR^4R^5$ to produce a compound in which $R^3$ represents hydrogen;

(d) alkylating or acylating the corresponding compound in which $R^3$ represent hydrogen to produce a compound in which $R^3$ represents alkyl or a group of formula $-\overset{O}{\overset{\|}{C}}-R^7$;

(e) reacting a 5-haloisoxazole of formula

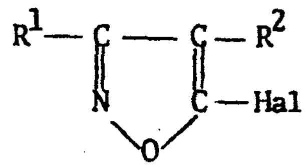

VI

where Hal represents a halogen atom, with a urea of formula

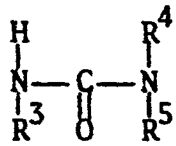

;

(f) reacting the corresponding compound in which $R^3$ and $R^5$ each represent a hydrogen atom with a halide of formula Hal-L-Hal where Hal represents a halogen atom and L represents alkylene, optionally unsaturated, optionally interrupted by one or more atoms selected from nitrogen, oxygen and sulphur atoms, and optionally substituted, to produce a compound in which $R^3$ and $R^5$ together represent L;

(g) salifying the 5-isoxazolyl urea to produce a salt of the

5-isoxazolyl urea; or

(h) desalifying a salt of the 5-isoxazolyl urea to produce the 5-isoxazolyl urea itself.

9. A compound claimed in any one of claims 1-7 prepared by a process claimed in claim 8.

10. A pesticidal or plant growth regulant composition comprising a compound which is a 5-isoxazolyl urea of formula

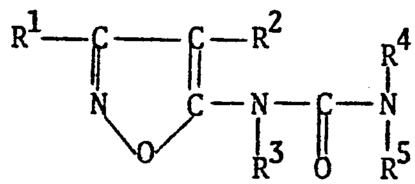

I

or a salt thereof,

wherein $R^1$ represents a hydrogen or halogen atom or a nitro, amino substituted amino, alkyl, substituted alkyl, cycloalkyl, aryl substituted aryl, aralkyl, or substituted aralkyl, group, or a group of formula $-XR^6$ or $-COOR^{15}$ in which X represents oxygen, sulphur, sulphinyl or sulphonyl, $R^6$ represents alkyl or aryl, and $R^{15}$ represents a hydrogen atom or an alkyl, substituted alkyl, aryl, substituted aryl, or cycloalkyl, group;

$R^2$ represents cyano, nitro, carboxy, carboxy ester, carboxyamide, carboxyamide whose amide group is substituted, or a group of formula $-\overset{O}{\overset{\|}{C}}-N\big)$ in which $N\big)$ represents a 5-7 ring membered heterocyclic radical which optionally may be substituted;

$R^3$ represents hydrogen, alkyl or a group of formula $-\overset{O}{\overset{\|}{C}}-R^7$ in which $R^7$ represents alkyl, aryl, alkoxy or alkylthio;

$R^4$ represents hydrogen, alkyl or cycloalkyl;

$R^5$ represents hydrogen, alkyl, cycloalkyl, alkoxy, aryl or substituted aryl; or

$R^3$ and $R^5$ together represent alkylene, optionally unsaturated, optionally interrupted by one or more atoms selected from nitrogen, oxygen and sulphur atoms, and optionally substituted,

together with at least one material selected from carriers, surface active agents, inorganic salts, herbicidal antidotes, fertilizers, other pesticides and other plant growth regulants.

11. A composition according to claim 10 which contains a surface active agent.

12. A method of combating pests at a locus infested or liable to be infested by them or of regulating the growth of a desired plant at a locus at which the plant is growing or is to grow, which method comprises applying to the locus a pest-combating amount or a plant growth regulant amount of a compound which is a 5-isoxazolyl urea of formula

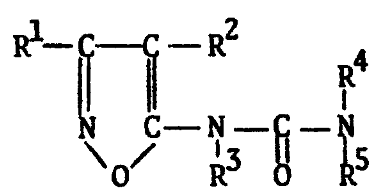

I

or a salt thereof,

wherein $R^1$ represents a hydrogen or halogen atom or a nitro, amino, substituted amino, alkyl, substituted alkyl, cycloalkyl, aryl,

substituted aryl, aralkyl, or substituted aralkyl, group, or a group of formula $-XR^6$ or $-COOR^{15}$ in which X represents oxygen, sulphur, sulphinyl or sulphonyl, $R^6$ represents alkyl or aryl, and $R^{15}$ represents a hydrogen atom or an alkyl, substituted alkyl, aryl, substituted aryl, or cycloalkyl, group;

$R^2$ represents cyano, nitro, carboxy, carboxy ester, carboxyamide, carboxyamide whose amide group is substituted, or a group of formula 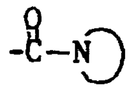 in which 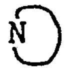 represents a 5-7 ring membered heterocyclic radical which optionally may be substituted;

$R^3$ represents hydrogen, alkyl or a group of formula $-\overset{O}{\overset{\|}{C}}-R^7$ in which $R^7$ represents alkyl, aryl, alkoxy or alkylthio;

$R^4$ represents hydrogen, alkyl or cycloalkyl;

$R^5$ represents hydrogen, alkyl, cycloalkyl, alkoxy, aryl or substituted aryl; or

$R^3$ and $R^5$ together represent alkylene, optionally unsaturated, optionally interrupted by one or more atoms selected from nitrogen, oxygen and sulphur atoms, and optionally substituted.

13. A method according to claim 12 wherein weeds are selectively combated in a crop.

14. A method according to claim 13 wherein the crop is a cereal crop or potatoes.

15. A method according to claim 13 or 14 wherein 0.05-10 kg of the compound are applied per hectare.

16. Propyl 5-amino-3-methylisoxazole-4-carboxylate, isopropyl 5-amino-3-methylisoxazole-4-carboxylate, butyl 5-amino-3-methylisoxazole-4-carboxylate, ethyl 5-amino-3-ethylisoxazole-4-carboxylate, propyl 5-amino-3-ethylisoxazole-4-carboxylate, isopropyl 5-amino-3-ethylisoxazole-4-carboxylate, or ethyl 5-amino-3-propylisoxazole-4-carboxylate.

17. A process for preparing propyl 5-amino-3-methylisoxazole-4-carboxylate, isopropyl 5-amino-3-methylisoxazole-4-carboxylate, butyl 5-amino-3-methylisoxazole-4-carboxylate, ethyl 5-amino-3-ethylisoxazole-4-carboxylate, propyl 5-amino-3-ethylisoxazole-4-carboxylate, isopropyl 5-amino-3-ethylisoxazole-4-carboxylate, or ethyl 5-amino-3-propylisoxazole-4-carboxylate,

which process comprises

(a) reacting the corresponding cyano derivative of formula

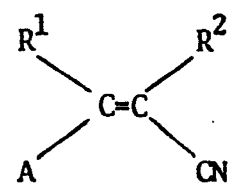

VII

wherein $R^1$ represents methyl, ethyl or propyl, $R^2$ represents propoxycarbonyl, isopropoxycarbonyl, butoxycarbonyl or ethoxycarbonyl and A represents hydroxy, halogen, amino, $-OR^9$, $-SR^9$, $-SOR^9$ or $-SO_2R^9$ where $R^9$ represents alkyl of 1-6 carbon atoms, with hydroxylamine;

(b) reacting a salt of formula $M^{\oplus}\underset{CN}{\overset{\ominus}{C}}HR^2$ where $M^{\oplus}$ represents a cation with a nitrile oxide of formula $R^1\overset{\oplus}{C}=N-\overset{\ominus}{O}$ or with the corresponding hydroxamyl chloride of formula $R^1\overset{Cl}{C}=NOH$, where $R^1$ and $R^2$ are as defined above; or

(c) reacting the 5-haloisoxazole of formula

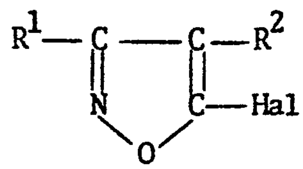

VI

where Hal represents a halogen atom, with ammonia, where $R^1$ and $R^2$ are as defined above.

18. A compound claimed in claim 16 prepared by a process claimed in claim 17.

European Patent Office

# EUROPEAN SEARCH REPORT

0002881

Application number

EP 78 300 497.1

## DOCUMENTS CONSIDERED TO BE RELEVANT

| Category | Citation of document with indication, where appropriate, of relevant passages | Relevant to claim |
|---|---|---|
| - | DE - A - 2 436 179 (SHIONOGI)<br>* page 2, 3 *<br>& GB - A - 1 471 743 | 8 |
| P | & US - A - 4 062 861<br>-- | |
| - | DE - A - 2 544 367 (SHIONOGI)<br>* page 2 bis 4 *<br>& GB - A - 1 463 439<br>& US - A - 4 028 376<br>-- | 8 |
| - | US - A - 3 547 940 (DU PONT)<br>* column 2 *<br>-- | 8 |
| - | US - A - 3 743 498 (DU PONT)<br>* claims 1, 10 *<br>-- | 10,12 |
| - | US - A - 3 917 632 (HOFFMANN-LA ROCHE)<br>* claim 1 *<br>---- | 17 |

**CLASSIFICATION OF THE APPLICATION (Int. Cl.²)**

C 07 D 261/14
A 01 N 5/00
C 07 D 261/18
A 01 N 9/22//
C 07 D 413/04
C 07 D 413/06
C 07 D 417/04

**TECHNICAL FIELDS SEARCHED (Int.Cl.²)**

A 01 N 5/00
A 01 N 9/22
C 07 D 261/14
C 07 D 261/18
C 07 D 413/04

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 08-02-1979 | FROELICH |

EPO Form 1503.1 06.78